(19) Europäisches Patentamt
European Patent Office
Office européen des brevets

(11) EP 2 678 009 B1

(12) **EUROPEAN PATENT SPECIFICATION**

(45) Date of publication and mention of the grant of the patent:
**01.11.2017 Bulletin 2017/44**

(21) Application number: **12750124.5**

(22) Date of filing: **23.02.2012**

(51) Int Cl.:
***A61K 31/145*** (2006.01) ***A61K 31/185*** (2006.01)
***A61K 31/198*** (2006.01) ***A61P 25/16*** (2006.01)

(86) International application number:
**PCT/CA2012/050106**

(87) International publication number:
**WO 2012/113079 (30.08.2012 Gazette 2012/35)**

(54) **CYSTAMINE ANALOGUES FOR THE TREATMENT OF PARKINSON'S DISEASE**

CYSTAMINANALOGA ZUR BEHANDLUNG VON MORBUS PARKINSON

ANALOGUES DE CYSTAMINE POUR LE TRAITEMENT DE LA MALADIE DE PARKINSON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2011 CA 2732440**
**23.02.2011 US 201161445577 P**

(43) Date of publication of application:
**01.01.2014 Bulletin 2014/01**

(73) Proprietor: **Université Laval**
**Québec, Québec G1V 0A6 (CA)**

(72) Inventors:
- **CICCHETTI, Francesca**
**Québec, Québec G2C 0C6 (CA)**
- **ROUILLARD, Claude**
**Québec, Québec G1C 6R3 (CA)**
- **CALON, Frédéric**
**Québec, Québec G1S 1S6 (CA)**

(74) Representative: **Gevers & Orès**
**41 avenue de Friedland**
**75008 Paris (FR)**

(56) References cited:
**WO-A2-2004/069175** **WO-A2-2008/143876**
**US-A1- 2009 076 166**

- **GIBRAT C ET AL: "Beneficial effects of cystamine following MPTP-induced parkinsonism in rodents", SOCIETY FOR NEUROSCIENCE ABSTRACT VIEWER AND ITINERARY PLANNER, vol. 40, 2010, XP9178759, & 40TH ANNUAL MEETING OF THE SOCIETY-FOR-NEUROSCIENCE; SAN DIEGO, CA, USA; NOVEMBER 13 -17, 2010**
- **GIBRAT C ET AL: "P2.130 Neuroprotective mechanisms of cystamine in a murine model of Parkinson's disease", PARKINSONISM AND RELATED DISORDERS, ELSEVIER SCIENCE, OXFORD, GB, vol. 15, 1 December 2009 (2009-12-01), pages S124-S125, XP026805921, ISSN: 1353-8020 [retrieved on 2009-12-01]**
- **WU J -Y ET AL: "G-CSF and taurine in neuroprotection and Parkinson's disease", SOCIETY FOR NEUROSCIENCE ABSTRACT VIEWER AND ITINERARY PLANNER, vol. 40, 2010, XP9178758, & 40TH ANNUAL MEETING OF THE SOCIETY-FOR-NEUROSCIENCE; SAN DIEGO, CA, USA; NOVEMBER 13 -17, 2010**
- **BOUSQUET ET AL.: 'Cystamine metabolism and brain transport properties: clinical implications for neurodegenerative diseases' JOURNAL OF NEUROCHEMISTRY vol. 114, no. 6, September 2010, pages 1651 - 1658, XP055122606**
- **GIBRAT ET AL.: 'Potential of cystamine and cysteamine in the treatment of neurodegenerative diseases' PROGRESS IN NEURO-PSYCHOPHARMACOLOY & BIOLOGICAL PSYCHIATRY vol. 35, no. 2, March 2011, pages 380 - 389, XP028156601**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GIBRAT ET AL.: 'Cystamine prevents MPTP-induced toxicity in young adult mice via the up-regulation of the brain-derived neurotrophic factor' PROGRESS IN NEURO-PSYCHOPHARMACOLOGY & BIOLOGICAL PSYCHIATRY vol. 34, no. 1, February 2010, pages 193 - 203, XP026832715
- TREMBLAY ET AL.: 'Neuroprotective effects of cystamine in aged parkinsonian mice' NEUROBIOLOGY OF AGING vol. 27, no. 6, June 2006, pages 862 - 870, XP024993215
- STACK ET AL.: 'Therapeutic attenuation of mitochondrial dysfunction and oxidative stress in neurotoxin models of Parhinson's disease' BIOCHIMICA ET BIOPHYSICA ACTA vol. 1782, no. 3, March 2008, pages 151 - 162, XP022487203
- SUN ET AL.: 'Effects of cysteamine on MPTP-induced dopaminergic neurodegeneration in mice' BRAIN RESEARCH vol. 1335, June 2010, pages 74 - 82, XP055122611

## Description

Current treatments for Parkinson's disease (PD) are largely symptomatic and do not prevent neuronal degeneration underlying the progression of the disease. The properties of cystamine in Parkinson's disease and in Huntington's disease have been studied in various animal models. In animal models of Huntington's disease (HD), cystamine has shown neuroprotective effects by prolonging life span and decreasing motor symptoms of mice carrying the Huntington's disease gene (Dedeoglu *et al.* 2002; Karpuj *et al.* 2002). *In vitro* and *in vivo* evidence have shown the capacity of cystamine to inhibit transglutaminase, an enzyme implicated in protein aggregates such as the mutated form of the huntingtin protein (Green 1993; Jeitner *et al.* 2005; Wang *et al.* 2005). The increase in brain levels of the brain derived neurotrophic factor (BDNF) have also been pinpointed as one of the key elements of this neuronal protective effect (Borrell-Pages *et al.* 2006). High dose of cystamine delivered through drinking water attenuates oxidative stress and deleterious effect of 1-methyl-4-phenyl-1,2,3,6-tetrahydropyridine (MPTP) on mitochondrial functions (Stack *et al.* 2008). The effects of cystamine and/or cysteamine have been reported in a MPTP mouse model of Parkinson's disease (Sun *et al.* 2010, Tremblay *et al.* 2006; Stack *et al.* 2008; Gibrat *et al.* 2010).

The metabolism of cystamine generates several intermediates including not only cysteamine, but also hypotaurine and taurine. Cystamine and cysteamine are both organic compounds and were initially described as radioprotectants (Bacq and Beaumariage 1965). Although cysteamine is the decarboxylated form of cysteine, the main source results from its constitutive production by all tissues via the degradation of coenzyme A (Pitari *et al.* 1992), which is involved in metabolic processes notably in the generation of ATP through the Krebs cycle (Leonardi *et al.* 2005). Although cysteine is a common constituent of most proteins (Lee *et al.* 2004), basal cysteine plasma levels are usually low because its thiol is susceptible to oxidation and leads to the disulfide derivative cystine.

Cysteamine, the reduced form of cystamine (2-aminoeth-anethiol) is approved for the treatment of cystinosis, a childhood disorder which causes renal failure through intracellular accumulation of cystine (Dohil *et al.* 2009). Because cysteamine has shown significant efficacy in mice models of Huntington's disease (Borrell-Pages *et al.* 2006) and its safety has been documented, the molecule is currently in development for patients suffering from this disorder (Dubinsky and Gray 2006).

A preliminary trial with cysteamine bitartrate (CYSTAGON®) was recently undertaken in Huntington's disease patients, in part to establish a safe therapeutic dose (Dubinsky and Gray, 2006). Nine Huntington's disease patients were enrolled in this single-center, open-label phase I clinical trial. Subjects received cysteamine treatment of 10 mg/kg per day with a weekly increase of an additional 10 mg/kg per day up to a maximum dose of 70 mg/kg or until the development of intolerable side effects (nausea and motoric impairment). The trial concluded that a dose of 20 mg/kg per day of cysteamine was tolerable in people suffering from Huntington's disease (Dubinsky and Gray, 2006). However, clinical efficacy was not demonstrated. Even if they are not entirely transposable to humans, studies carried out in Huntington's disease animal models showed that much higher doses of cystamine or cysteamine were required to achieve a significant therapeutic effect. Furthermore, although cysteamine can cross the blood brain barrier (BBB), it takes larger doses of cystamine or cysteamine (i.p. or p.o.) to detect a variation in cysteamine or its metabolites in the brain (Bousquet *et al.,* 2010). The efficacy of cystamine and cysteamine to modify the progression of Parkinson's disease, as well as their brain transport properties are unknown.

Existing therapies for Parkinson's disease are mainly designed for symptom management and so far there is no treatment available to attenuate the progression of the disease. There is therefore a need for the development of therapeutic agents that can modify the rate of progression of Parkinson's disease.

The inventors have demonstrated, for the first time, that cystamine analogues can be used to modify the progression of Parkinson's disease.

The invention is defined in the claims; the following disclosure is subject to this definition. The present disclosure provides a method for modifying the progression of Parkinson's disease, the method comprising administering a therapeutically effective amount of at least one cystamine analogue, a pharmaceutically acceptable salt thereof, a composition or combination of the invention to a patient in need thereof.

The present disclosure provides the use of a therapeutically effective amount of at least one cystamine analogue, a pharmaceutically acceptable salt thereof, a composition or combination of the invention for modifying the progression of Parkinson's disease in a patient in need thereof.

The present disclosure provides the use of a therapeutically effective amount of at least one cystamine analogue or a pharmaceutically acceptable salt thereof as a neurorescuing agent and/or as a neurorestoration agent for modifying the progression of Parkinson's disease in a patient.

The present invention disclosure a combination or a pharmaceutical composition for modifying the progression of Parkinson's disease comprising at least one cystamine analogue or a pharmaceutically acceptable salt thereof and comprising cysteine or a pharmaceutically acceptable salt thereof.

The present disclosure provides a combination or a pharmaceutical composition for modifying the progression of Parkinson's disease comprising at least one cystamine analogue or a pharmaceutically acceptable salt thereof as a

neurorescuing agent and/or neurorestoration agent and comprising cysteine or a pharmaceutically acceptable salt thereof.

In still another aspect, there is provided the use of a cystamine analogue, or a pharmaceutically acceptable salt thereof, a composition or combination of the invention for the manufacture of a medicament as described herein.

## BRIEF DESCRIPTION OF FIGURES:

**Figure 1:** Beneficial effects of cystamine on nigral tyrosine hydroxylase positive neurons.

(a) Stereological cell counts of tyrosine hydroxylase (TH)-positive neurons in the subtantia nigra pars compacta (SNpc) revealed a significant decrease in the total number of TH-positive neurons in MPTP mice treated with saline, as compared to saline + saline animals ($p < 0.001$). (a) Pre and Post-MPTP cystamine treated mice demonstrated a similar number of TH-positive neurons as saline treated animals. (b) Photomicrographs of the SNpc showing an elevated number (comparable to saline) of Cresyl stained and TH-positive neurons in the saline and post-MPTP cystamine treated mice as compared to MPTP saline treated mice. The table in (c) recapitulates the Cresyl and TH stereological cell counts. Lower panels illustrate time lines of the pre and post-treatment schedules. Values are expressed as means ± S.E.M. Statistical analyses were performed using one-way ANOVA. Significant difference with the saline + saline group: *** = $p < 0.001$. Significant difference with the MPTP + saline group: # = $p < 0.05$; ## = $p < 0.01$; ### = $p < 0.001$. Scale bar in (b) = 400 μm, inset = 25 μm. Abbreviations: Pre-Tx (pre-MPTP cystamine treatment); Post-Tx (post-MPTP cystamine treatment).

**Figure 2:** Beneficial effect of cystamine on nigral Nurr1 mRNA expression.

(a) Densitometric measurements of Nurr1 mRNA levels (a gene involved in the expression and maintenance of the dopamine (DA) phenotype) in the SNpc revealed that the levels of the 3 control groups (saline + saline; saline + cystamine Pre-Tx, saline + cystamine Post-Tx) and MPTP animals treated with cystamine were similar, while Nurr1 mRNA levels in MPTP animals treated with saline were significantly decreased ($p < 0.01$). (b) Photomicrographs at the level of the SNpc (see arrow) illustrates the normal levels of Nurr1 mRNA in the control and post-MPTP cystamine treated mice as compared to MPTP saline treated mice (b). Values are expressed as means ± S.E.M. Statistical analyses were performed using one-way ANOVA. Significant difference with the saline + saline group: ** = $p < 0.01$. Significant difference with the MPTP + saline group: # = $p < 0.05$; ## = $p < 0.01$. Scale bar in b = 1 mm.

**Figure 3:** Beneficial effect of cystamine on nigral DAT positive cells.
The expression of the DA transporter (DAT) mRNA was also revealed by in situ hybridization. (a) Stereological cell counts of DAT expressing cells in the SNpc showed a significant decrease in the total number of neurons in MPTP mice treated with saline, as compared to saline + saline animals ($p < 0.001$). (a) Pre and post-MPTP cystamine treated mice showed a comparable number of DAT-positive cells as saline treated animals. (b) Photomicrographs of the SNpc represent DAT mRNA expressing cells. The inset depicts the DAT mRNA autoradiography before emulsion (measured by densitometry). The table in (c) recapitulates the stereological cell counts and densitometric measurements of DAT mRNA expression in the SNpc. Values are expressed as means ± S.E.M. Statistical analyses were performed using one-way ANOVA. Significant difference with the saline + saline group: * = $p < 0.05$; *** = $p < 0.001$. Significant difference with the MPTP + saline group: # = $p < 0.05$; ## = $p < 0.01$. Scale bar in b = 400 μm, inset = 500 μm.

**Figure 4:** Time course of nigral TH positive cell loss in the subacute MPTP model.

(a) Stereological cell counts of TH-positive neurons in the SNpc revealed a significant decrease in the total number of TH-positive neurons 7 and 14 days following the last MPTP injection as compared to saline ($p < 0.01$) and the 1-day post-MPTP group, which only showed a tendency toward a decreased number of neurons ($p = 0.063$).
(b) Photomicrographs of the SNpc depict a reduced number of Cresyl stained and TH-positive neurons in the 7 and 14-day post-MPTP groups. The table in (c) recapitulates the Cresyl and TH stereological cell counts. Values are expressed as means ± S.E.M. Statistical analyses were performed using one-way ANOVA. Significant difference with the saline group: ** = $p < 0.01$. Scale bar in b = 400 μm.

**Figure 5:** Time course of decreases in Nurr1 and DAT mRNA expression in the subacute MPTP model.
Densitometric measurements of (a) Nurr1 and (b) DAT mRNA expression showed significantly decreased levels of both DA markers in the SNpc beginning at 24 h post MPTP treatment ($p < 0.01$ and $p < 0.05$ respectively). Values are expressed as means ± S.E.M. Statistical analyses were performed using one-way ANOVA. Significant difference with the control group: ** = $p < 0.01$. * = $p < 0.05$.

**Figure 6:** Time course of nigral DA apoptotic process in the subacute MPTP model.
Western blot analysis of (a) BAX and (b) Bcl-2 protein levels in the ventral mesencephalon. (c) The BAX/Bcl2 ratio is increased significantly 24 h after the last MPTP injection ($p < 0.05$) suggesting that, with this specific regimen of MPTP delivery, an apoptotic process has already begun at this time. Values are expressed as means ± S.E.M. Statistical analyses were performed using one-way ANOVA. Significant difference with the control group: * = $p < 0.05$.

**Figure 7:** Increased levels of brain cysteamine. Cerebral cysteamine (b) and cysteine (c) levels measured by HPLC coupled with fluorescence detection. Molecular structures and HPLC elution profiles of a standard solution of cysteamine (2) and cysteine (1) are represented in (a). Cysteamine is significantly increased in response to a single cystamine i.p. injection of 50 mg/kg in mice killed 1 h following the injection, as compared with vehicle mice killed at the same time point ($p < 0.05$) (b). The 200 mg/kg dose also provokes a significant increase of cysteamine 1 h and 3 h following the cystamine injection ($p < 0.01$) (b). Cysteine cerebral levels are stable regardless of doses and perfusion times (c). Data are expressed as means (nmol/mg of protein) ± S.E.M. *$p < 0.05$; **$p < 0.01$.

**Figure 8:** Constant levels of brain hypotaurine and taurine. Cerebral hypotaurine (b) and taurine (c) concentrations measured by HPLC coupled with UV detection. Molecular structures and HPLC elution profiles of a standard solution containing 1 ng/mL of taurine (1) and hypotaurine (2) are represented in (a). Stable brain measures of hypotaurine (b) and taurine (c) were observed. Data are expressed as means (nmol/mg of protein) ± S.E.M.

**Figure 9:** Increased cysteamine brain uptake in the presence of cysteine. Demonstration of cysteamine and cysteine brain uptake using *in situ* cerebral perfusion technique and quantification by HPLC method. Schematic illustration of *in situ* cerebral perfusion method (a). A catheter is directly inserted into the right internal carotid artery to ensure 100% of the perfusate reaches the right hemisphere after proper ligatures (blue vessels) (a). Both cysteine (b) and cysteamine (c) can cross the BBB as demonstrated by the high clearance coefficient of each molecule (μL/g/s). When co-perfused, cysteine and cysteamine clearance coefficients increase significantly. Data are expressed as means ± S.E.M. (μL/g/s) *$p < 0.05$.

**Figure 10:** Cystamine rescues dopaminergic neurons in the unilateral striatal 6-OHDA mouse model of Parkinson's disease. Panel (E) illustrates the time course of the experiment. All mice were subjected to unilateral intrastriatal injection of 6-OHDA (4 ug) or equivalent volume of vehicle (sham). Three days later, during the ongoing DAergic degenerative process, the mice received a treatment of 10 mg/kg cystamine (or saline) daily for 14 days. The mice were transcardially perfused 24 hours after the last cystamine injection and brains processed for post-mortem analyses. (A) Stereological cell count of TH-positive neurons in the SNpc revealed a significant 72% decrease in the total number of TH-positive neurons in 6-OHDA mice treated with saline, as compared to sham + saline animals ($p < 0.001$). 6-OHDA mice treated with cystamine displayed only a 27% decrease in the total number of TH-positive neurons as compared to sham + cystamine animals ($p < 0.05$). The 6-OHDA + saline group was significantly different of the 6-OHDA + cystamine group ($p < 0.001$). (B) and (C) Densitometric measurements for Nurr1 and DAT mRNA levels in the SNpc respectively. These 2 additional markers of DAergic integrity revealed the same pattern as observed for the TH staining in (A), confirming the neurorescue properties of cystamine. (D) The measurements of striatal DA content performed by HPLC showed significantly decreased levels of DA only in 6-OHDA mice treated with saline as compared to control mice ($p<0.01$). Values are expressed as means ± S.E.M. Statistical analyses were performed using one-way ANOVA. *= $p < 0.05$; ** = $p < 0.01$; *** = $p < 0.001$.

**Figure 11:** Cystamine reverses behavioral impairments induced by striatal 6-OHDA lesions in mice. Panel (D) illustrates the time course of the experiment. All mice were subjected to unilateral intrastriatal injection of 6-OHDA (4 ug) or equivalent volume of vehicle (sham). Three weeks later, when the lesion was stable and had reached maximum degeneration, the mice received a treatment of 10 mg/kg cystamine (or saline) daily for 6 weeks. Throughout the experiment, mice were evaluated with 3 different behavioral tests at 3 distinct time points: prior the initiation of cystamine treatment (3 weeks post-surgery), 6 weeks and 9 weeks post-lesion. The behavioral tests were chosen to assess the sensorimotor asymmetries indicative of the unilateral DAergic lesion extent. (A) Cumulative apomorphine-induced contralateral rotations were measured with a rotometer apparatus. The unilateral 6-OHDA lesion induced an increase of contralateral rotations 3, 6 and 9 weeks post-lesion significantly attenuated by the cystamine

treatment 6 and 9 weeks post-surgery ($p < 0.05$). (B) The stepping test revealed a decrease in the percentage of adjusting steps for the contralateral forepaw (compared to ipsilateral). (C) A limb-use asymmetry is also shown for the cylinder test in which 6-OHDA-lesioned mice showed a significant decrease in the percentage of contralateral contacts at 3 weeks, 6 weeks and 9 weeks post-surgery compared to sham mice. This asymmetry was not visible for 6-OHDA-lesioned mice treated with cystamine at 6 and 9 weeks post-lesion. Values are expressed as means ± S.E.M. Statistical analyses were performed using one-way ANOVA. *= $p < 0.05$; ** = $p < 0.01$; *** = $p < 0.001$.

**Figure 12:** Cystamine restores some aspects of the dopaminergic nigral system and changes striatal catecholaminergic contents and dynamics in the unilateral striatal 6-OHDA mouse model of Parkinson's disease. Panel (D) illustrates the time course of the experiment. As shown in Figure 11, all mice were subjected to unilateral intrastriatal injection of 6-OHDA (4 ug) or equivalent volume of vehicle (sham) and the cystamine (or saline) treatment began 3 weeks later for a period of 6 weeks. The mice were transcardially perfused 24 hours after the last cystamine injection and brains processed for post-mortem analyses. (A) Stereological cell count of TH-positive neurons in the SNpc revealed a significant 93% decrease in the total number of TH-positive neurons in 6-OHDA mice treated with saline, as compared to sham + saline animals ($p < 0.001$). 6-OHDA mice treated with cystamine displayed only a 65% decrease in the total number of TH-positive neurons as compared to sham + cystamine animals ($p < 0.001$). The 6-OHDA + saline group is significantly different from the 6-OHDA + cystamine group ($p < 0.01$). (B) The DA levels of cystamine measured by HPLC in 6-OHDA lesioned mice were decreased by 88% and 84% for saline and cystamine treated animals respectively ($p < 0.001$). Cystamine did not significantly restore DA content in the lesioned striatum. (C) However cystamine exerted a strong tendency to normalise the DA turnover as compared to 6-OHDA mice treated with saline, which significantly correlates with behavioural lesion-evaluation tests (data not shown). Values are expressed as means ± S.E.M. Statistical analyses were performed using one-way ANOVA. *= $p < 0.05$; ** = $p < 0.01$; *** = $p < 0.001$.

## Uses and Methods

In one embodiment, the cystamine analogues and pharmaceutically acceptable salts thereof can be used as neurorescue and/or neurorestorative agents. This neurorescue/neurorestorative activity can be distinguished from the activity of a neuroprotective agent.

As used herein, "a neuroprotective agent" can protect the remaining 'healthy' neurons from the degenerative process. Therefore it can be appreciated that a neuroprotective agent could be administered at the time of diagnosis.

As used herein, "a neurorescuing agent" can stop the neurodegenerative process on neurons that are injured, but not dead, with or without functional recovery. Therefore it can be understood that a neurorescuing agent must be given as early as possible, but can be administered after the diagnosis of PD.

As used herein, "a neurorestorative agent" can re-establish function by functional and/or structural restoration and regeneration of the injured neurons. It can therefore be appreciated that a neurorestorative agent is highly relevant for clinical use in PD since it can show maximal efficacy after diagnosis.

In a further embodiment, the cystamine analogues and pharmaceutically acceptable salts thereof can be used to modify the progression of Parkinson's disease.

In one embodiment, "modifying the progression of Parkinson disease" is characterized by a) a reduction of the neurodegenerative process by an anti-apoptotic action on neurons that are injured, but not dead, with or without functional recovery; and/or b) functional and/or structural restoration and regeneration.

In a further embodiment, "modifying the progression of Parkinson disease" is characterized by one of the following mechanisms:

a) a reduction of the neurodegenerative process by an anti-apoptotic action on neurons that are injured, but not dead, with or without functional recovery; and/or

b) functional and/or structural restoration and regeneration of the injured neurons, and/or

c) promoting neurogenesis.

In yet another embodiment, the progression of Parkinson's disease is quantified by the Total Unified Parkinson's Disease Rating Scale (Total UPDRS) score, an increase in the Total UPDRS score represents progression of Parkinson's disease symptoms, and the increment of the increase in Total UPDRS score over a period of time represents the rate of progression of Parkinson's disease. See for example:Goetz CG, Tilley BC, Shaftman SR, Stebbins GT, Fahn S, Martinez-Martin P, Poewe W, Sampaio C, Stern MB, Dodel R et al. (2008) Movement Disorder Society-sponsored revision of the Unified Parkinson's Disease Rating Scale (MDS-UPDRS): scale presentation and clinimetric testing

results. Mov Disord 23:2129-2170.

In yet another embodiment of this method, the period of time is 12, 24, or 36 weeks after initiation of administration of cystamine analogue or a pharmaceutically acceptable salt thereof.

As used herein, stages of a Parkinson's disease patient is described by Hoehn and Yahr in following five distinct stages depending on the symptoms (Hoehn M M, Yahr M D, Parkinsonism: onset, progression and mortality. Neurology 1967, 17:427-42).

Stage I: (mild or early disease): Symptoms affect only one side of the body.

Stage II: Both sides of the body are affected, but posture remains normal.

Stage III: (moderate disease): Both sides of the body are affected, and there is mild imbalance during standing or walking. However, the person remains independent.

Stage IV: (advanced disease): Both sides of the body are affected, and there is disabling instability while standing or walking. The person in this stage requires substantial help.

Stage V: Severe, fully developed disease is present. The person is restricted to a bed or chair.

As used herein, an "early stage Parkinson's disease patient" is a Parkinson's disease patient at Stage I or II of the Parkinson's Disease as defined by Hoehn and Yahr, and who does not require symptomatic anti-Parkinsonian therapy. In one embodiment, such Parkinson's disease patient does not require symptomatic treatment for at least the next 9 months. An early stage Parkinson's disease patient may be identified as such by performing relevant testing.

In an embodiment, the patient is an early stage Parkinson's disease patient.

In yet another embodiment, the early stage Parkinson's disease patient is a patient having a UPDRS total score of less than 30; less than 25; less than 23; less than 21; or less than 20.

In yet another embodiment, the Parkinson's disease patient is a Stage II, III or IV patient according to Hoehn and Yahr rating.

In yet another embodiment, the Parkinson's disease patient is a Stage III or IV patient according to Hoehn and Yahr rating.

In yet another embodiment, the Parkinson's disease patient is a Stage III, or IV patient according to Hoehn and Yahr rating.

In yet another embodiment, the Parkinson's disease patient is a Stage III according to Hoehn and Yahr rating.

The present disclosure relates to uses or methods for:

reducing fatigue in a Parkinson's disease patient;
reducing the severity of non-motor symptoms in a Parkinson's disease patient;
reducing the functional decline in a Parkinson's disease patient;
reducing the clinical progression of the disease; or
slowing the clinical progression of the disease.

The subject disclosure yet further provides a method of treating a patient exhibiting Parkinson's disease, comprising the identification of patients exhibiting Parkinson's disease, and periodically administering to the patient so identified an amount of cystamine analogue or a pharmaceutically acceptable salt thereof, or a composition or combination of the invention effective to treat the patient.

The present disclosure relates to a method of slowing or reducing the progression of Parkinson's disease in a patient comprising administering to the patient a therapeutically effective amount of at least one cystamine analogue or a pharmaceutically acceptable salt thereof, a composition or combination of the invention.

The present disclosure relates to the use of a therapeutically effective amount of at least one cystamine analogue or a pharmaceutically acceptable salt thereof, a composition or combination of the invention for slowing or reducing the progression of Parkinson's disease in a patient.

The subject disclosure further provides the use of a therapeutically effective amount of cystamine analogue or a pharmaceutically acceptable salt thereof or a composition or combination of the invention for slowing clinical progression of Parkinson's disease in a Parkinson's disease patient.

The subject disclosure further provides relates to a method for slowing clinical progression of Parkinson's disease in a Parkinson's disease patient comprising administering to the patient a therapeutically effective amount of cystamine analogue or a pharmaceutically acceptable salt thereof or a composition or combination of the invention.

The subject disclosure yet further provides the use of a therapeutically effective amount of cystamine analogue

or a pharmaceutically acceptable salt or a composition or combination of the invention for reducing the severity of non-motor symptoms in a Parkinson's disease patient.

The subject disclosure yet further provides a method for reducing the severity of non-motor symptoms in a Parkinson's disease patient comprising administering to the patient a therapeutically effective amount of cystamine analogue or a pharmaceutically acceptable salt or a composition or combination of the invention.

The subject disclosure yet further provides a method of treating a patient having stage II, III, IV of Parkinson's disease (according to Hoehn and Yahr rating), comprising the identification of patients exhibiting Parkinson's disease, and periodically administering to the patient so identified an amount of cystamine analogue or a pharmaceutically acceptable salt thereof, or a composition or combination of the invention effective to treat the patient.

The subject disclosure further provides a cystamine analogue or a pharmaceutically acceptable salt thereof, or a composition or combination of the invention for use in treating a patient exhibiting early signs of Parkinson's disease.

The subject disclosure yet further provides a pharmaceutical composition comprising a pharmaceutically effective amount of cystamine analogue or a pharmaceutically acceptable salt thereof, or a composition or combination of the invention for use in reducing the rate of progression of Parkinson's disease in an early stage Parkinson's disease patient.

The subject disclosure yet further provides the use of a therapeutically effective amount of cystamine analogue or a pharmaceutically acceptable salt thereof or a composition or combination of the invention for treating a patient exhibiting early signs of Parkinson's disease.

The subject disclosure yet further provides a method of treating a patient exhibiting early signs of Parkinson's disease, comprising the identification of patients exhibiting early signs of Parkinson's disease, and periodically administering to the patient so identified an amount of cystamine analogue or a pharmaceutically acceptable salt thereof, or a composition or combination of the invention effective to treat the patient.

The subject disclosure further provides cystamine analogue or a pharmaceutically acceptable salt thereof or a composition or combination of the invention for use in reducing the fatigue in an early stage Parkinson's disease patient.

The subject disclosure yet further provides the use of a therapeutically effective amount of cystamine analogue or a pharmaceutically acceptable salt thereof or a composition or combination of the invention for reducing the fatigue in an early stage Parkinson's disease patient.

The subject disclosure yet further provides a method of reducing the fatigue in an early stage Parkinson's disease patient, comprising identifying a patient to be an early stage Parkinson's disease patient, and periodically administering to the patient so identified an amount of cystamine analogue or a pharmaceutically acceptable salt thereof effective to reduce fatigue.

The subject disclosure yet further provides a method of reducing the severity of non-motor symptoms in an early stage Parkinson's disease patient, comprising identifying a patient to be an early stage Parkinson's disease patient, and periodically administering to the patient so identified an amount of cystamine analogue or a pharmaceutically acceptable salt thereof or a composition or combination of the invention effective to reduce the severity of non-motor symptoms.

The subject disclosure further provides a method of reducing fatigue in an early stage Parkinson's disease patient, comprising periodically administering to an early stage Parkinson's disease patient an amount of cystamine analogue or a pharmaceutically acceptable salt thereof or a composition or combination of the invention effective to reduce fatigue.

The subject disclosure further provides a method of reducing severity of non-motor symptoms in an early stage Parkinson's disease patient, comprising periodically administering to an early stage Parkinson's disease patient an amount of cystamine analogue or a pharmaceutically acceptable salt thereof or a composition or combination of the invention effective to reduce the severity of non-motor symptoms.

The subject disclosure further provides a method of slowing clinical progression of Parkinson's disease in a Parkinson's disease patient comprising periodically administering to the Parkinson's disease patient an amount of cystamine analogue or a pharmaceutically acceptable salt thereof, or a composition or combination of the invention effective to slow clinical progression of Parkinson's disease in the patient.

The subject disclosure further provides cystamine analogue or a pharmaceutically acceptable salt thereof, or a composition or combination of the invention for use in reducing the rate of progression of Parkinson's disease in an early stage Parkinson's disease patient.

The subject disclosure further provides cystamine analogue or a pharmaceutically acceptable salt thereof, or a composition or combination of the invention for use in reducing the functional decline in an early stage Parkinson's disease patient.

**Pharmaceutical Compositions and Cystamine Analogues**

The cystamine analogue is cysteamine, cystamine, taurine or hypotaurine or a pharmaceutically acceptable

salt thereof.

In a further aspect, the cystamine analogue is cystamine or cysteamine or a pharmaceutically acceptable salt thereof.

In a further aspect, the cystamine analogue is cysteamine bitartrate.

In a further aspect, the cystamine analogue is cysteamine hydrochloride.

In a further aspect, the cystamine analogue is cystamine dihydrochloride.

In another aspect, there is provided a pharmaceutical composition comprising at least one of such cystamine analogue or a pharmaceutically acceptable salt thereof and at least one pharmaceutically acceptable carrier or excipient.

In another aspect, there is provided a combination comprising a cystamine analogue or a pharmaceutically acceptable salt thereof and one or more additional agents such as bromocriptine, benzotropine, levodopa, ropinirole, pramipexole, rotigotine, cabergoline, entacopone, tolcapone, amantidine, selegiline and rasagiline.

In still another aspect, there is provided the use of a cystamine analogue, or a pharmaceutically acceptable salt thereof, a composition or combination of the invention for the manufacture of a medicament for modifying the progression of Parkinson's disease in a patient.

In accordance with a further embodiment, the compounds of the present invention are represented by the following formulae:

$NH_2$-$(CH_2)_2$-SH

Cysteamine

$NH_2$-$(CH_2)_2$-S-S-$(CH_2)_2$-$NH_2$

Cystamine

$NH_2$-$(CH_2)_2$-S$(O_2)$-OH

Taurine

$NH_2$-$(CH_2)_2$-S(O)-OH

Hypotaurine

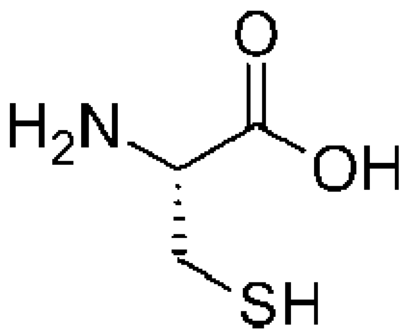

L-cysteine

or pharmaceutically acceptable salts thereof.

Cystamine analogues or pharmaceutically acceptable salt thereof can be obtained by methods well-known in the art. The compounds are available from different sources, for example, from Sigma-Aldrich, St. Louis, MO. USA.

In one embodiment, the present invention provides a pharmaceutical composition comprising at least one cystamine analogue or pharmaceutically acceptable salt thereof described herein, and further comprising at least one additional agent wherein the additional agent is cysteine.

In one embodiment, the present invention provides a pharmaceutical composition comprising at least cystamine analogue or pharmaceutically acceptable salt thereof described herein, and further comprising at least one additional agent wherein the additional agent is L-cysteine.

In another embodiment, there is provided a combination comprising at least one cystamine analogue described herein and one or more additional agents.

In one embodiment, wherein the additional agent is cysteine.

In one embodiment, wherein the additional agent is L-cysteine.

In one embodiment, the cystamine analogue and cysteine are present in a ratio 10:1 to 1:10 of cystamine analogue and cysteine respectively. In a further embodiment, the cystamine analogue and cysteine are present in a ratio of 1:1.

In one combination embodiment, the cystamine analogue and additional agent are administered or suited to be used sequentially.

In another combination embodiment, the cystamine analogue and additional agent are administered or suited to be used simultaneously.

The combinations referred to above may conveniently be presented for use in the form of a pharmaceutical formulation and thus pharmaceutical formulations comprising a combination as defined above together with a pharmaceutically acceptable carrier therefore comprise a further aspect of the invention.

The individual components for use in the method of the present invention or combinations of the present invention may be administered either sequentially or simultaneously in separate or combined pharmaceutical formulations.

In one embodiment, the present invention provides the use of a compound, composition or combination as described herein for the manufacture of a medicament.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. The single optical isomer or enantiomer can be obtained by method well known in the art, such as chiral HPLC, enzymatic resolution and chiral auxiliary.

In one embodiment, where applicable, the cystamine analogues or cysteine are provided in the form of a single stereoisomer at least 75%, 85%, 90%, 95%, 97% and 99% free of the corresponding stereoisomers.

There is also provided pharmaceutically acceptable salts of the cystamine analogues or cysteine. By the term pharmaceutically acceptable salts of compounds are meant those derived from pharmaceutically acceptable inorganic and organic acids and bases. Examples of suitable acids include hydrochloric, hydrobromic, sulphuric, nitric, perchloric, fumaric, maleic, phosphoric, glycollic, lactic, salicylic, succinic, toleune-p-sulphonic, tartaric, acetic, trifluoroacetic, citric, methanesulphonic, formic, benzoic, malonic, naphthalene-2-sulphonic and benzenesulphonic acids. Other acids such as oxalic, while not themselves pharmaceutically acceptable, may be useful as intermediates in obtaining the cystamine analogues and their pharmaceutically acceptable acid addition salts.

Salts derived from amino acids are also included (e.g. L-arginine, L-Lysine).

Salts derived from appropriate bases include alkali metals (e.g. sodium, lithium, potassium) and alkaline earth metals (e.g. calcium, magnesium).

A reference hereinafter to the cystamine analogues or cysteine includes that compound and its pharmaceutically acceptable salts.

In one embodiment, the salt is a bitartrate salt.

In one embodiment, the salt is a hydrochloride salt.

With regards to pharmaceutically acceptable salts, see also the list of FDA approved commercially marketed salts listed in Table I of Berge et al., Pharmaceutical Salts, J. of Phar. Sci., vol. 66, no. 1, January 1977, pp. 1-19, the disclosure of which is incorporated herein by reference.

It will be appreciated by those skilled in the art that the compounds can exist in different polymorphic forms. As known in the art, polymorphism is an ability of a compound to crystallize as more than one distinct crystalline or "polymorphic" species. A polymorph is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds.

It will further be appreciated by those skilled in the art that the compounds in accordance with the present invention can exist in different solvate forms, for example hydrates. Solvates of the cystamine analogues or cysteine may also form when solvent molecules are incorporated into the crystalline lattice structure of the compound molecule during the crystallization process.

Unless otherwise defined, all technical and scientific terms used herein have the same meaning as commonly understood by one of ordinary skill in the art to which this invention belongs. In addition, the materials, methods, and examples are illustrative only and not intended to be limiting.

For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

Additionally, unless otherwise stated, the cystamine analogues or cysteine depicted herein are also meant to include compounds that differ only in the presence of one or more isotopically enriched atoms. For example, the cystamine analogues or cysteine, wherein one or more hydrogen atoms are replaced deuterium or tritium, or one or more carbon atoms are replaced by a 13C- or 14C-enriched carbon are within the scope of this invention. Such compounds are useful, for example, as analytical tools, probes in biological assays, or compounds with improved therapeutic profile.

It will be appreciated that the amount of a cystamine analogues required for use in treatment will vary not only with the particular compound selected but also with the route of administration, the nature of the condition for which treatment is required and the age and condition of the patient and will be ultimately at the discretion of the attendant physician. In general however a suitable dose will be in the range of about 0.1 to about 750 mg/kg of body weight per day, for example, in the range of 0.5 to 60 mg/kg/day, or, for example, in the range of 1 to 20 mg/kg/day.

The desired dose may conveniently be presented in a single dose or as divided dose administered at appropriate intervals, for example as two, three, four or more doses per day.

The cystamine analogue is conveniently administered in unit dosage form; for example containing 5 to 2000 mg, 10 to 1500 mg, conveniently 20 to 1000 mg, most conveniently 50 to 700 mg of active ingredient per unit dosage form. In one embodiment, the cystamine analogue is conveniently administered in unit dosage form of 600 mg twice daily.

When cystamine analogues or pharmaceutically acceptable salts thereof are used in combination with a second therapeutic agent active against Parkinson's disease the dose of each compound may be either the same as or differ from that when the compound is used alone. Appropriate doses will be readily appreciated by those skilled in the art.

While it is possible that, for use in therapy, the cystamine analogues may be administered as the raw chemical it is preferable to present the active ingredient as a pharmaceutical composition. The invention thus further provides a pharmaceutical composition comprising the cystamine analogues or a pharmaceutically acceptable salt of the present invention thereof together with one or more pharmaceutically acceptable carriers therefore and, optionally, other therapeutic and/or prophylactic ingredients. The carrier(s) must be "acceptable" in the sense of being compatible with the other ingredients of the formulation and not deleterious to the recipient thereof.

Pharmaceutical compositions include those suitable for oral, rectal, nasal, topical (including buccal and sublingual), transdermal, vaginal or parenteral (including intramuscular, sub-cutaneous and intravenous) administration or in a form suitable for administration by inhalation or insufflation. The compositions may, where appropriate, be conveniently presented in discrete dosage units and may be prepared by any of the methods well known in the art of pharmacy. All methods include the step of bringing into association the active with liquid carriers or finely divided solid carriers or both and then, if necessary, shaping the product into the desired composition.

Pharmaceutical compositions suitable for oral administration may conveniently be presented as discrete units such as capsules, cachets or tablets each containing a predetermined amount of the active ingredient; as a powder or granules; as a solution, a suspension or as an emulsion. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The cystamine analogues may also be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g., sterile, pyrogen-free water, before use.

For topical administration to the epidermis, the cystamine analogues may be formulated as ointments, creams or lotions, or as a transdermal patch. Such transdermal patches may contain penetration enhancers such as linalool, carvacrol, thymol, citral, menthol and t-anethole. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or colouring agents.

Compositions suitable for topical administration in the mouth include lozenges comprising active ingredient in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Pharmaceutical compositions suitable for rectal administration wherein the carrier is a solid are for example presented as unit dose suppositories. Suitable carriers include cocoa butter and other materials commonly used in the

art, and the suppositories may be conveniently formed by admixture of the active compound with the softened or melted carrier(s) followed by chilling and shaping in moulds.

Compositions suitable for vaginal administration may be presented as pessaries, tampons, creams, gels, pastes, foams or sprays containing in addition to the active ingredient such carriers as are known in the art to be appropriate.

For intra-nasal administration the compounds or combinations may be used as a liquid spray or dispersible powder or in the form of drops. Drops may be formulated with an aqueous or non-aqueous base also comprising one more dispersing agents, solubilizing agents or suspending agents. Liquid sprays are conveniently delivered from pressurized packs.

For administration by inhalation the compounds or combinations are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount.

Alternatively, for administration by inhalation or insufflation, the compounds or combinations may take the form of a dry powder composition, for example a powder mix of the compound and a suitable powder base such as lactose or starch. The powder composition may be presented in unit dosage form in, for example, capsules or cartridges or e.g. gelatin or blister packs from which the powder may be administered with the aid of an inhalator or insufflator.

When desired the above described compositions adapted to give sustained or modified release of the active ingredient may be employed. Examples of cysteamine formulation are described for example in US publication 20090076166.

The present inventors have surprisingly found that cystamine has beneficial effects in parkinsonian animals when administered prior and after to the toxin MPTP capable of triggering the pathology. The present inventors have determined that cystamine can overturn an initiated neurodegenerative process. As described in the examples, mice were subacutly intoxicated with MPTP following a 5-day regimen of 7 i.p. injections of 20 mg/kg and administered 10 mg/kg of cystamine i.p. daily either 1) 2 days before the start of MPTP injections or 2) 24 hours after the last MPTP dose, and which continued for 14 days post injury. At the end of the study, post-mortem analyses were performed to assess the state of the dopaminergic (DAergic) system, more particularly targeted in PARKINSON'S DISEASE. The present inventors found surprisingly that i.p. administration of cystamine (10 mg/kg/day) to MPTP-treated mice commencing after the impairment of the nigrostriatal system (24 hours after the MPTP treatment), induced a significant recovery of the number of nigral DAergic neurons, as assessed by stereological count of TH-immunoreactive cells, ($p<0.05$), of the number of DAT mRNA expressing cells ($p<0.05$) as well as nigral Nurr1 mRNA levels ($p<0.05$). The present inventors have also determined that cystamine rescues dopaminergic neurons in the unilateral striatal 6-OHDA mouse model of Parkinson's disease. Further as shown in the examples herein, cystamine reverses behavioral impairments induced by striatal 6-OHDA lesions in mice and restores some aspects of the dopaminergic nigral system and changes striatal catecholaminergic contents and dynamics in the unilateral striatal 6-OHDA mouse model of Parkinson's disease. The present inventors found that the role of cystamine compounds is not only limited to preserving the existing neurons. The compounds can also reverse a prompted apoptotic process and thus rescue damaged neurons from undergoing degeneration.

Without being bound to any specific theory, the present inventors believe that cysteamine is the key neuroactive compound following systemic administration of cystamine. Among the molecules investigated through HPLC measurements, which included cysteamine, cysteine, hypotaurine and taurine, cysteamine was found to be the only one significantly increased in brains of naïve mice following a single i.p. injection of 50 mg/kg and 200 mg/kg. In contrast, cysteine, hypotaurine and taurine levels remained unchanged or under the threshold of detection. In addition, the present inventors have demonstrated that cysteamine crosses BBB in significant amount in vivo. These observations provide important information pertaining to the neuropharmacology of cysteamine and further support its clinical relevance.

The BBB is a major obstacle to the clinical application of a vast majority of potentially neuroactive compounds and must be taken into account when determining which metabolite of cystamine exerts its therapeutic effect. Amino acid transporters are well-studied BBB components and comprise leucine, alanine, serine, or cysteine-preferring systems (Wade and Katzman 1975; Sershen and Lajtha 1979). Cysteine has been recognized to use the leucine-preferring system to cross the BBB (Wade and Brady 1981). The capacity of taurine to cross the BBB has also been described in rats utilizing ISCP and seemingly involves an endothelial cell sodium and chloride-dependant influx system (Benrabh et al. 1995). The mechanism by which cysteamine, on the other hand, can cross the BBB requires further investigations. Here, a quantitative and highly sensitive technique was employed to study BBB transport of cysteamine and cysteine. This was performed without compromising the physical or functional integrity of the BBB and by bypassing peripheral metabolism processes associated with systemic administration. The inventors demonstrated the capacity of cysteamine and cysteine to reach the brain in significant amount. Cysteamine brain uptake was further facilitated by the addition of cysteine in the perfusate.

In the foregoing and in the following examples, all temperatures are set forth uncorrected in degrees Celsius;

and, unless otherwise indicated, all parts and percentages are by weight.

The following examples can be repeated with similar success by substituting the generically or specifically described reactants and/or operating conditions of this invention for those used in the preceding examples.

## EXAMPLES:

### EXAMPLE 1: Effect of cystamine following MPTP-induced parkinsonism in rodents

***Animals***

Young adult (9-week old, 25 grams) male C57BL/6 mice were purchased from Charles River Laboratories (Montreal, QC, Canada). Animals were housed 4 per cage under standard conditions with free access to food and water, randomized and handled under the same conditions by one investigator. All experiments were performed in accordance with the Canadian Council on Animal Care and were approved by the Institutional Committee of the Centre Hospitalier de l'Université Laval (CHUL, Quebec, Canada). Throughout the experiment, the health status of all mice included in the study was closely monitored for weight loss or other signs of health-related issues. All efforts were made to minimize animal pain and discomfort.

***Administration of MPTP***

Mice received 7 i.p. injections, twice on the first 2 days of the experimental protocol at an interval of 12 hrs and once a day on 3 subsequent days, of either saline 0.9% or MPTP-HCl (20 mg/kg free base; Sigma, St. Louis, MO) dissolved in saline 0.9% prepared fresh (Tremblay *et al.,* 2006; Gibrat *et al.,* 2007; Gibrat *et al.,* 2010).

***Cystamine treatments***

Beneficial effects of cystamine in parkinsonian mice (cystamine dihydrochloride, Sigma, St. Louis, MO) were evaluated with a dose of 10 mg/kg dissolved in sterile saline 0.9% and prepared fresh for daily i.p. injection 1 hr before MPTP administration. The choice of dose and regime of administration was based on our previous findings (Tremblay et al., 2006; Gibrat et al., 2010). The first injection of cystamine was administered either 1) 2 days before the start of MPTP injections (pre-treatment) or 2) 24 hours after the last MPTP dose (post-treatment), and the treatment continued daily for 14 days post injury.

This study was divided into 2 distinct experiments.

***Experiment no.1. Neurorescue properties of cystamine in MPTP lesioned mice***

The effects of cystamine on the MPTP toxicity were studied in the following experimental groups: Group I, Saline + Saline; Group II, Saline + Cystamine post-treatment; Group III; Saline + Cystamine pre-treatment; Group IV, MPTP + Saline; Group V, MPTP + Cystamine post-treatment, Group VI, MPTP + Cystamine pre-treatment. In total, 96 mice (n=16 per group) were utilized, monitored daily for weight variation, and ultimately sacrificed by perfusion 24 hrs after the last cystamine (or vehicle) injection.

***Experiment no.2. Time course of nigral DAergic neuronal death induced by a subacute MPTP treatment***

For this experiment, a total of 72 mice were used and divided into 6 groups (n=12 per group). Group I, II and III received a subacute treatment of MPTP while Group IV, V and VI were administered with saline 0.9%. Group I and IV were sacrificed 24 h, Group II and V: 7 days and Group III and VI: 14 days following the last MPTP (or saline) injection.

***Perfusion and tissue processing***

Animals were sacrificed under deep anesthesia with ketamine/xylazine (Vetalar, Bioniche, Belleville, ON/Rompun, Bayer, Toronto, ON) and perfused according to two methods in RNAse free conditions:

Experiment 1. All mice were subjected to intracardiac perfusion with RNAse free 0.1 M phosphate-buffered saline (PBS). After intracardiac perfusion, brains were collected and the two hemispheres were separated. The left hemisphere was post-fixed in 4% paraformaldehyde (PFA) for 48 hrs and transferred to 20% sucrose in 0.1 M PBS for cryoprotection. Coronal brain sections of 25 μm thickness were cut onto a freezing microtome (Leica Microsystems, Montreal, QC) and serially collected in antifreeze solution (monophosphate sodium monobasic 0.2 M, monophosphate sodium dibasic 0.2 M, ethylene glycol 30%, glycerol 20%) and kept at -20 °C until use. Sections from the left hemisphere were utilized for additional immunohistochemistry and *in situ* hybridization protocols. The right hemispheres were snap-frozen in 2-methyl-butane and then stored at -80 °C until cryostat dissection for HPLC and western-blot (WB) analyses.

Experiment 2. In this experiment, the 2 hemispheres of each animal were snap-frozen and used for HPLC and WB analyses. The 5 remaining mice of each group were perfused intracardiacaly by RNAse free saline (0.9%) followed by 4% PFA, pH 7.4. After intracardiac perfusion, brains were collected and post-fixed in 4% PFA for 24 hrs and transferred to 20% sucrose in 0.1 M PBS for cryoprotection. Brains were cut into coronal sections of 25 μm thickness. These sections were used for immunohistochemistry and *in situ* hybridization required for the completion of experiment 2.

***Catecholamine quantification by HPLC***

Striatal DA, 3,4-dihydroxyphenylacetic acid (DOPAC) and homovanilic acid (HVA) concentrations were measured by HPLC coupled with electrochemical detection (Calon *et al.,* 2001; Calon *et al.,* 2003). Each striatal sample comprised ten 20 μm thick cryostat sections of the structure ranging between levels +1.145 and +1.345 (Allen, 2008; Lein *et al.,* 2007). Two hundred μl of perchloric acid (0.1 N; J. T. Baker) was added to each sample, which were homogenized and centrifuged (13000 xg) to generate a supernatant. Fifty μl of supernatant from striatal tissues were directly injected into the chromatograph system consisting in a Waters 717 plus autosampler automatic injector, a Waters 1525 binary pump equipped with an Atlantis dC18 (3 μl) column, a Waters 2465 electrochemical detector, and a glassy carbon electrode (Waters Limited, Lachine, QC, Canada). Electrochemical potential was set at 10 nA. The mobile phase consisted of 47.8 mM $NaH_2PO_4$, 0.9 mM sodium octyl sulfate (J. T. Baker), 0.4 mM EDTA, 2 mM NaCl, and 8% methanol (J. T. Baker) at pH 2.9 and was delivered at 1.0 ml/min. Peaks were identified using Breeze software (Waters). HPLC quantifications were normalized to protein concentrations, as determined with a bicinchoninic acid (BCA) protein assay kit (Pierce, Rockford, IL, USA).

***TH immunohistochemistry***

For assessment of DAergic neuronal-loss, immunohistochemistry against the enzyme TH was performed as previously described (Tremblay *et al.,* 2006; Gibrat *et al.,* 2009). Briefly, free-floating sections, after several washes and blocking preincubation, were incubated overnight at 4ºC with a rabbit anti-TH (Pel-Freez, Rogers, AR; 1:5000). Sections were then incubated for 1 hr at room temperature (RT) in a solution containing biotinylated goat anti-rabbit IgG (Vector Laboratories, Burlington, ON; 1:1500) and subsequently placed in a solution containing avidin-biotin peroxidase complex (ABC Elite kit; Vector Laboratories, Burlington, ON) for 1 hr at RT. Finally, the reaction was developed in 3,3' diaminobenzidine tetrahydrochloride (DAB) solution (Sigma, St. Louis, MO) and 0.1% of 30% hydrogen peroxide (Sigma, St. Louis, MO) at RT. Other sections were treated as above except that the primary antibody was omitted from the incubation medium. These sections remained virtually free of immunostaining and served as negative controls. Following the DAB reaction, sections were mounted on gelatin-coated slides and counterstained with cresyl violet (Sigma, St. Louis, MO). All sections were finally air-dried, dehydrated in ascending grades of ethanol, cleaned in xylene, and coverslipped with DPX mounting media (Electron Microscopy Science, Hatfield, PA).

***In situ hybridization for Nurr1 and DAT***

A specific [$^{35}$S]UTP-labeled complementary RNA (cRNA) probe was used to assess tissue mRNA levels of *Nurr1,* a nuclear receptor associated with the DAergic system (Zetterstrom *et al.,* 1997). The cRNA probe for *Nurr1* stems from a 403 bp (gene bank accession number: 1504-1907 NM_013613) EcoRI-BamHI fragment of a full-length mouse *Nurr1* cDNA subcloned into pBluescript SK+ and linearized with Xba I.

The DAT probe, a 2238 bp length fragment, was cloned into pBluescript II SK+ plasmid. Linearization was made with NotI enzyme. Antisense probe was synthesized with [35S]UTP and T7 RNA polymerase.

Sense probes were also generated for these markers and no specific signal was obtained (data not shown). Brain sections were hybridized following the procedures described below and previously published protocols (Beaudry et al., 2000; Cossette *et al.,* 2004; Lapointe *et al.,* 2004).

This *in situ* protocol was conducted in RNAse free conditions. Slices were mounted onto Snowcoat X-tra™ slides (Surgipath, Winnipeg, Canada) and stored under vacuum overnight before use. Brain sections were fixed in 4% PFA pH 7.4 at RT for 20 min. Pre-treatment was made with various consecutive baths (PBS 0.1 M twice 5 min, proteinase K 0.1 μg/ml 10 min at 37°C, acetylation bath (0.25% acetic anhydride, triethanolamine 0.1 M) 10 min, twice for 5 min in standard saline citrate (SSC) (0.3 M NaCl, 30 mM sodium citrate)). Successive baths of ethanol solutions (30%, 60%,

100%, 100%; 3 min each) were performed for dehydration. *In situ* hybridization of the riboprobes on tissue sections was performed at 58 °C overnight in a standard hybridization buffer (deionised formamid 50%, sodium chloride 5 M, Tris 1 M, EDTA 0.5 M, Denhart's solution 50X, dextran sulfate 50%, tRNA 10 mg/mL, DTT 1 M, $^{35}$S coupled 2X10$^{6}$ cpm/μl probe). Post-treatment was conducted using different successive baths: SSC 4X (30 min), removing coverslips, SSC 2X twice (5 min), RNase A 20 μg/mL (1 hr) at 37 °C, milliQ water twice (15 sec), SSC 2X (15 min), SSC 0.5X (30 min) at 60 °C, SSC 0.1X (30 min) at 60 °C, SSC 0.1X (5 min) at RT. Repetitive baths of ethanol solutions (30%, 60%, 100%, 100%; 3 min each) were used for further dehydration. Tissue sections were then placed against BiomaxMR (Kodak, New Haven, CT) radioactive sensitive films. Autoradiograms were developed following a 72 h exposure for *Nurr1* and 5 h exposure for DAT.

Deffating was performed with 4 baths of ethanol, 2 baths of xylene and 3 baths of ethanol. Following these steps, slides were dipped in NTB emulsion (Kodak, New Haven, CT) melted at 42 °C, air-dried for 4h and stored in the dark for 5 days at 4 °C. The emulsion was then developed (3.5 min) in D-19 developer (Kodak, New Haven, CT), rinsed in deionised water and fixed (5 min) in Rapid Fixer solution from Kodak. Slides were rinsed in deionised water for 1 h and then coloured. Coloration was performed using thionine (1 min), followed by water and ethanol dips then 3 ethanol (1 min) and 3 xylene baths (3 min). Slides were coverslipped with DPX mounting media.

***Western-blot analyses***

Samples were homogenized in 8 volumes of lysis buffer (150 mM NaCl, 10 mM NaH2PO4, 1% (v/v) Triton X-100, 0.5% SDS, and 0.5% sodium deoxycholate) containing a cocktail of protease inhibitors (Roche, Mississauga, ON, Canada) and phosphatase inhibitors (Sigma, St-Louis, MO, USA). Samples were sonicated (3 × 10 sec) and centrifuged at 100,000 *g* for 20 min at 4 °C. The supernatant was collected and stored at -80 °C. The protein concentration in each fraction was determined with a bicinchoninic acid protein assay kit. Twenty μg of total protein per sample were added to Laemmli loading buffer and heated to 95 °C for 5 min. Samples were then loaded and subjected to SDS-polyacrylamide (12%) gel electrophoresis. Proteins were electroblotted onto 0.45 μm Immobilon PVDF membranes (Millipore, Billerica, MA, USA) and blocked in 5% nonfat dry milk and 1% BSA in 1X PBS for 1 h. Membranes were immunoblotted with primary antibodies, rabbit anti-TH (Pel-Freez; 1:5,000), rabbit ant-BAX (Cell signalling technology; Danvers, MA; 1:1,000), rabbit anti-Bcl2 (Cell signalling technology; 1:1,000), mouse anti-actin (ABM Inc, Richmond, BC, Canada; 1:10,000), and with appropriate secondary antibodies, goat anti-rabbit or anti-mouse (Jackson Immunoresearch, West Grove, PA; 1:100,000) followed by the addition of chemiluminescence reagents (KPL, Mandel Scientific, Guelph, ON, Canada). Band intensities were quantified using a ImageQuant Las 4000 Digital Imaging System (Science Lab 2003 Image Gauge Software version 4.2, Fujifilm, New Haven, CT).

***Densitometric measurements of Nurr1 and DAT mRNA levels***

Levels of autoradiographic labeling were quantified by computerized densitometry. Digitized brain images and their analyses were made with the same equipment as mentioned above. Optical density of the autoradiograms was translated in μCi/g of tissue using $^{14}$C radioactivity standards (ARC 146-$^{14}$C standards, American Radiolabeled Chemicals Inc., St. Louis, MO). *Nurr1* and DAT mRNA levels were measured in the substantia nigra compacta (SNc) using similar antero-posterior levels for all sections. The average labeling for each SNc level was calculated from 3 adjacent brain sections of the same mouse. Background intensities taken from white areas of the substantia nigra reticulata (SNr) devoid of *Nurr1* or DAT mRNA levels were subtracted from every measurement.

***Stereological quantification of TH-immunoreactive neurons***

The loss of DAergic neurons was determined by stereological counts of TH-immunoreactive cells (identifiable somas) under bright-field illumination. Every 10th section through the SNc was analyzed using Stereo investigator software (MicroBrightfield, Colchester, VT, USA) attached to an E800 Nikon microscope (Nikon Canada Inc., Mississauga, ON, Canada). After delineation of the SNc at low magnification (4X objective), a point grid was overlaid onto each section. For the most rostral level of SNc analyzed (bregma -3.08mm), the SNc was delineated by the visible boundaries with the medial terminal nucleus. For the intermediate (bregma - 3.28mm) and most caudal levels of SNc analyzed (bregma -3.58mm), the structure was demarcated by the exit of the 3rd cranial nerve. Immunostained cells were counted by the optical fractionator method at higher magnification (20X objective). The counting variables were as follow: distance between counting frames (150 μm X 150 μm), counting frame size (75 μm) and guard zone thickness (1 μm). Cells were counted only if they did not intersect forbidden lines. The optical fractionator (Glaser and Glaser, 2000) method was used to count TH-positive (TH- and cresyl violet-positive) and TH-negative (cresyl violet-positive only) cells. Stereological cell counts were performed blindly by two independent investigators. Note that the analyses of the TH-immunoreactive profiles were restricted to the SNc and thus excluded the ventral tegmental area (VTA).

***Statistical analyses and image preparation***

All analyses are expressed as group mean ± S.E.M. Data pertaining to experiment no. 1 and 2 were assessed by two-way ANOVA. When the two-way ANOVA yielded non-significant interaction terms, the data were further analyzed for significance using the Tukey post-hoc multiple comparison test. In all cases, a P value of less than 0.05 was considered to be significant. Photomicrographs were taken by Picture Frame software (Microbrightfield) attached to a E800 Nikon microscope (Nikon Instruments, Toronto, ON). Images were finalized for illustration using Adobe Photoshop CS3.

**RESULTS**

**The effects of cystamine on the DAergic system**

**Neuroprotective effects of cystamine in a subactute MPTP mouse model**

Endpoint histological evaluation was conducted in all mice comprised in this study to investigate the beneficial effects of cystamine using several specific markers related to the DA system. TH is the rate-limiting enzyme in DA biosynthesis and a marker for DA neurons. *Nurr1* is a transcriptional factor involved in the maintenance of the DAergic phenotype and the dopamine transporter, DAT, is a highly specific marker of pro DAergic nigrostriatal neurons and thus, their expression reflects the state of DAergic neuronal health.

The MPTP treatment generated a significant loss of TH-immunoreactive neurons that was associated with a concomitant loss of Nissl-stained neurons in the SNpc, consistent with a degeneration of DA neurons as opposed to a downregulation of TH expression ($p < 0.001$, Fig. 1). This was accompanied by a significant decrease in nigral *Nurr1* and DAT mRNA levels in the SNpc ($p < 0.01$, Fig. 2; $p < 0.001$, Fig. 3). Daily drug administration of 10 mg/kg cystamine started 2 days before the MPTP intoxication, confirming its neuroprotective action as revealed by the increase density of TH-immunoreactive cells in the SNpc ($p < 0.001$, Fig. 1), as compared to non-treated MPTP animals. Post-mortem analysis of the DA system in cystamine pretreated mice further demonstrated the normalization of *Nurr1* mRNA levels ($p < 0.01$, Fig. 2) as well as the density of SNpc neurons expressing DAT ($p < 0.01$, Fig. 3).

**Neurorescue potential of cystamine in a subactute MPTP mouse model**

The neurorescue properties of cystamine treatment were evaluated beginning 24 h after the last MPTP injection. In mice post-treated with 10 mg/kg of cystamine, MPTP-induced DAergic neurotoxicity was also significantly reduced. Mice treated with cystamine after the MPTP injury exhibited a significantly greater number of TH-positive and Nissl-positive neurons ($p < 0.01$, Fig. 1) as well as a higher level of *Nurr1* ($p < 0.5$, Fig. 2) and DAT ($p < 0.5$, Fig. 3) mRNA, comparable to those observed in non-treated MPTP mice.

Overall, evaluations of these three specific markers related to the DA system yielded similar patterns and showed the beneficial effects of a post-MPTP treatment of cystamine, not confining cystamine to neuroprotection but extending the properties of cystamine to neurorescue.

In order to conclude on the capacity of cystamine to not only prevent (neuroprotective) but also stop (neurorescue) the neurodegenerative process, the inventors undertook a study in order to define the time course of DA-related degeneration of the MPTP model used in these experiments.

**Time course of nigral DAergic cells degeneration induced by subacute MPTP administration**

Loss of TH-positive and Nissl-positive neurons varied between 20% and 27% in the MPTP groups sacrificed from day 1 to day 14 alter the last MPTP injection but was only statistically significant at day 7 and 14 compared to correspondent saline groups ($p < 0.01$, Fig. 4). Despite the absence of a significant TH-positive cell loss at day 1, a significant reduction in *Nurr1* and DAT mRNA levels in the SNpc ($p < 0.05$, Fig. 5) was observed, indicating some vulnerability of the DA neurons. Moreover, the pro- and anti-apoptotic proteins, BAX and Bc12, were respectively increased and decreased 24 h alter the last injection of MPTP as assessed by western-blot analyses ($p < 0.05$, Fig. 6). Taken together, these findings indicate that although the DAergic neurons have not begun to degenerate 24 hours alter the last injection of MPTP, they are engaged in the apoptotic pathway. Importantly, this support that the beneficial effect of cystamine is of neurorescuing nature.

**RESULTS**

**The effects of cystamine on the DAergic system**

**EXAMPLE 2: Cystamine metabolism and brain transport properties**

**Animals and cystamine administration**

Young adult (9-week old, 25 g) male C57BL/6 mice were purchased from Charles River Laboratories (Montreal, QC, Canada). Animals were housed four per cage under standard conditions with free access to food and water, randomized and handled under the same conditions by one investigator. All experiments were performed in accordance with the Canadian Council on Animal Care and were approved by the Institutional Committee of the Centre Hospitalier de l'Université Laval (CHUL). Throughout the experiment, the health status of all mice included in the study was closely monitored. To clearly identify the active intermediate following cystamine injection, as well as to understand its systemic and cerebral metabolism, a single intraperitoneal (i.p.) injection of cystamine was administered to normal adult C57BL/6 male mice using three different doses: 10, 50, and 200 mg/kg, as determined in prior publications (Tremblay *et al.* 2006; Gibrat *et al.* 2010). These doses were ultimately compared with saline injections. Cystamine was dissolved in sterile saline (0.9%) and injected 1, 3, 12, 24 and 48 h before killing. Animals were sacrificed under deep anesthesia with ketamine/xylazine and perfused via intracardiac infusion with 0.1 M phosphate-buffered saline. After intracardiac perfusion, brains were collected, snap-frozen in 2-methyl-butane and then stored at -80 °C until cryostat dissection for HPLC analyses. A total of 200 mice were assigned to this study (n = 10 per group).

**Cysteine and cysteamine HPLC measurements**

HPLC coupled to fluorescence detection was used in cysteine and cysteamine quantification of both sets of experiments: the dose-response study and *in situ* cerebral perfusion (ISCP) procedures. Frontal cortex were homogenized in 200 μL of $NaHCO_3$ and then centrifuged at 15 700 g (4 °C) for 20 min. Fifty μl of supernatant were directly derivatized with 30 μL of 4-fluoro-7-sulfamoylbenzo-furazan (ABD-F) reagent. The alkylation reaction was completed at 55 °C for 15 min and stopped with 4.9 μL HCl 12 N. After a 10-min centrifugation at 7500 g (4 °C), the supernatant were immediately injected into the chromatograph consisting of a Waters 717 plus autosampler automatic injector set at 4 °C, a Waters 1525 binary pump equipped with an Atlantis dC18 (3 μL; 3.9 x 150 mm) column, and a Waters 2487 Dual Absorbance detector (Waters limited, Lachine, QC, Canada). The excitation was set at 385 nm and emission at 515 nm. The mobile phase, which consisted in 2.5% methanol and 0.1 M ammonium acetate adjusted at pH 4.0, was delivered at 1 mL/min (Santa *et al.* 2006). Peaks were identified and quantified using Breeze software (Waters limited). HPLC quantifications were normalized to protein concentrations. Protein measurements were determined with a bicinchoninic acid protein assay kit (Pierce, Rockford, IL, USA) as described by the manufacturer's protocol.

**Taurine and hypotaurine HPLC measurements**

Taurine and hypotaurine were measured by HPLC coupled with UV detection. Supernatant from $NaHCO_3$ brain (bregma 1.54 to -0.58 mm) extracts (see section above for details) were directly derivatized with the reagent dansyl chloride (Sigma-Aldrich, St. Louis, MO, USA) based on modified published methods (Saller and Czupryna 1989; Calon *et al.* 1999). Briefly, 50 μL of dansyl chloride (1.2 mg/mL) and 50 μL of sample or standard solution were mixed and then incubated for 30 min at 90 °C. After a 10-min centrifugation at 7500 g (4 °C), supernatants were immediately injected into the chromatograph described above. Absorbance was set at 337 nm and the sensitivity at 0.5 absorbance unit full scale. The mobile phase consisted of a water-acetonitrile mixture (88.5-11.35% v/v) containing 0.15% (v/v) of phosphoric acid and was delivered at a rate of 0.8 mL/min. Results were obtained using the same method as described above.

***In situ* cerebral perfusion**

*In situ* cerebral perfusion (ISCP) was performed under deep anesthesia prompted by i.p. injection of a mixture of ketamine/xylazine (140/8 mg/kg) and as previously described (Dagenais *et al.* 2000; Ouellet *et al.* 2009). To ensure that 100% of the perfusate reached the BBB, the right common carotid artery was catheterized following ligation of the external branch (see Fig. 3a for schematic representation). The thorax was then opened, the heart removed and the perfusion immediately initiated at a flow rate of 2.5 mL/min. The perfusion solution consisted of bicarbonate buffered physiological saline: 128 mM NaCl, 24 mM $NaH\text{-}CO_3$, 4.2 mM KCl, 2.4 mM $NaH_2PO_4$, 1.5 mM $CaCl_2$, 0.9 mM $MgCl_2$ and 9 mM D-glucose. The solution was gassed with 95% $O_2$ and 5% $CO_2$ to obtain a pH of 7.4 and subsequently heated at 37 °C. In all experiments, a radiolabeled tracer ($^{14}C$-sucrose 0.3 μCi/mL) was co-perfused with cysteamine (259 μM)

and cysteine (165 μM), as a marker of BBB integrity and of the vascular volume. Four distinct groups of naïve mice (*n* = 3) were assessed in this study and were perfused with cysteine, cysteamine, both molecules or with the $^{14}C$-sucrose alone which served as the control.

The procedure was terminated by decapitation of the mouse after 60 s of perfusion. The right cerebral hemisphere was collected and the frontal cortex was dissected and rapidly frozen on dry ice for HPLC measurements of cysteine and cysteamine.

The remaining brain tissue of this hemisphere was digested in 2 mL of Solvable (Perkin-Elmer Life Sciences, Waltham, MA, USA)) at 50 °C for 48 h and mixed with 9 mL of Hisafe scintillation cocktail (Perkin-Elmer Life Sciences). Aliquots of the perfusion fluid were taken before adding the radiolabeled marker for HPLC quantification and alter its passage through the syringe and catheter for scintillation counting, at the end of each experiment, for the calculation of the brain transport coefficient (see equation below). $^{14}C$ isotope was counted in brain digest and in perfusate in a Wallac scintillation counter (Perkin-Elmer Life Sciences). The cysteine and cysteamine uptake clearance coefficients (Clup; μL/g/s) were calculated from the measured volume of distribution of cysteine or cysteamine, corrected with the vascular space determined with the $^{14}C$-sucrose. The vascular space was constant and under 20 μL/g. The following equation was used for final calculations, as previously described (Dagenais *et al.* 2000).

$$\text{Clup } (\mu l\ g^{-1}\ s^{-1}) = \frac{V_d}{T}, \text{ in which } V_d = \frac{X_{\text{cysteine}}}{C_{\text{cysteine perf}}} - \frac{X_{\text{sucrose}}}{C_{\text{sucrose perf}}}$$

$V_d$ (μL/g) represents the volume of distribution of the study compound, *T*(s) is time of perfusion, $X_{cysteine}$ (ng/mg of tissue) or $X_{sucrose}$ (dpm/g) is the quantity of cysteine or sucrose found in the frontal cortex or in the remaining tissue of the hemisphere, respectively. *C* is the concentration (ng/μL; dpm/mL) in the perfusion solution (cysteine serving as an example in the above equation).

**Data and statistical analyses**

All data are expressed as group mean ± S.E.M. Data were assessed by two-way ANOVAS and analyzed for significance using student's *t-test.* Each group was compared with the 0 mg/kg group of the associated time points (1, 3, 12, 24 or 48 h). For ISCP experiments, student's *t-test* were used to analyze significance. In all cases, a *p* value of less than 0.05 was considered to be significant.

**Results**

**General effect of cystamine administration**

Throughout the dose-response study, no death was reported and all mice displayed good health except for the 200 mg/kg cystamine group where mice displayed signs of hypothermia (shivering) and somnolence (closing of eyelids) for a period of approximately 2 h, as previously reported (Gibrat *et al.* 2010).

**Plasma and brain cysteine and cysteamine levels following cystamine administration**

To investigate the metabolites found in the plasma and brain of mice injected with a single cystamine i.p. dose, a highly sensitive HPLC method was utilized and allowed us to specifically measure cysteamine and cysteine through a thiol (-SH) derivatization using ABD-F compound prior to fluorescence detection (Fig. 7a). Cysteamine was undetectable in the plasma of cystamine-treated mice. Contrary to bodily expression, cerebral analyses of cysteamine and cysteine concentrations revealed a marked increased in brain cysteamine (Fig. 7b). This increase was observed for all three doses of cystamine administered and at each time point targeted by this dose-response study. Two-way ANOVA revealed significant differences for both factors; doses and time as well as a significant interaction between those two factors ($p < 0.0001$). *Post hoc* analyses revealed significant increases specifically at 1 h post-injection for the 50 mg/kg ($p < 0.05$) and 200 mg/kg ($p < 0.01$) doses. Cysteamine levels remained significantly elevated 3 h following cystamine administration ($p < 0.01$) and progressively diminished through 48 h, when compared with the saline group. Cystamine administration did not affect cysteine plasma (data not shown) or brain levels, even at the highest dose of 200 mg/kg (Fig. 7c). There were no indications of any significant changes in cysteine plasma and/or brain levels for any of the doses or time points studied.

**Plasma and brain hypotaurine and taurine levels following cystamine administration**

Hypotaurine is a major metabolite of cysteamine, which can, in part, generate taurine. To determine the concentration of these two molecules, a primary amino group derivatization wit dansyl chloride prior to UV detection was utilized (Fig. 8a). This reaction takes place in both aromatic and aliphatic amine, producing stable sulfonamide adducts and allowing the detection of both hypotaurine (Fig. 8a; compound 2) and taurine (Fig. 8a; compound 1) within the same method. Despite some variation between groups, no significant alteration of brain hypotaurine and taurine was observed for any of the three doses (10, 50 or 200 mg/kg) and as compared with control groups (Fig. 8b and c). There were no signs of brain accumulation of hypotaurine and taurine at any of the time points of killing (1, 3, 12, 24 and 48 h). Plasma levels remained under or close to the detection threshold.

**Cysteine facilitates cysteamine brain transport**

As a vast majority of endogenous and exogenous compounds are inactive in the CNS because they do not cross the BBB, the inventors investigated cysteamine and cysteine brain uptake. Using ISCP, the inventors measured cysteamine and cysteine blood-brain transport parameters by directly infusing the brain via the carotid artery (Dagenais *et al.* 2000; Ouellet *et al.* 2009) (Fig. 9a). Both cysteine and cysteamine crossed the BBB, as observed by their brain transport coefficient (Clup), which corresponded to 4.39 $\pm$ 0.47 and 0.15 $\pm$ 0.02 μL/g/s, respectively (Fig. 9b and c). In comparison, a routinely used CNS drug, like morphine, displays a Clup of 0.3 μL/g/s, whereas highly diffusible drugs like diazepam or fatty acids display a Clup which reaches up to 40 μL/g/s (Bourasset *et al.* 2003; Ouellet *et al.* 2009). Interestingly, co-perfusion of cysteine and cysteamine potentiated their brain uptake. Indeed, significant increase of the Clup of cysteamine (+133%; $p < 0.05$) and of cysteine (+59%; $p < 0.05$) were measured when both compounds were injected simultaneously. Hypotaurine and taurine, which have been shown to cross the BBB, were not re-evaluated (Benrabh *et al.* 1995).

**EXAMPLE 3 Neurorescue and neurorestorative properties of cystamine following intrastriatal administration of 6-OHDA**

**Material and method**

***Animals***

Young adult (9-week old, 25 grams) male C57BL/6 mice were purchased from Charles River Laboratories (Montreal, QC, Canada). Animals were housed 4 per cage under standard conditions with free access to food and water, randomized and handled under the same conditions by one investigator. All experiments were performed in accordance with the Canadian Council on Animal Care and were approved by the Institutional Committee of the Centre Hospitalier de l'Université Laval (CHUL). Throughout the experiment, the health status of all mice included in the study was closely monitored for weight loss or other signs of health-related issues. All efforts were made to minimize animal pain and discomfort.

***6-OHDA and cystamine treatment***

This protocol was based on the unilateral stereotaxic intra-striatal injection of 6-hydroxydopamine (6-OHDA). Intrastriatal administration of 6-OHDA creates a progressive and retrograde degenerative changes in DA neurons of the substantia nigra (Bjorklund et al., 1997; Costantini et al., 2001). Mice were anaesthetized using isoflurane and placed onto a stereotactic frame adapted for mice. 6-OHDA was dissolved at a concentration of 2 μg/μl in 0.9% saline and 0.02% ascorbic acid and 2 μl was injected in the right striatum at a rate of 0.5 μl/min. The needle was left in place for 3 min after the injection before retraction. The injection was performed using an Hamilton syringe according to the stereotaxic coordinates: AP: +0.04 cm, ML: -0.18 cm, DV: -0.31 cm (corresponding to the atlas of Allan P. 2008).

To serve as controls, other mice were subjected to the same surgical procedures, but only 2 μl of solvent (0.9% saline and 0.02% ascorbic acid) was injected in the same coordinates. The study was further divided into 2 distinct experiments:

1) The neurorescuing effects of cystamine (cystamine dihydrochloride, Sigma, St. Louis, MO) in 6-OHDA leisoned mice. For this experiment, the first i.p. injection of 10 mg/kg cystamine was administered 3 days post surgery and the treatment continued daily for 14 days.

2) The neurorestorative effects of cystamine (cystamine dihydrochloride, Sigma, St. Louis, MO) in 6-OHDA lesioned

mice. The treatment of cystamine began 3 weeks post surgery, when the dopaminergic (DAergic) lesion is known to be stable and has reached its peak. The treatment was continued daily for 6 weeks.

The effects of cystamine on the 6-OHDA toxicity were studied in the following experimental groups: Group I, Sham + Saline; Group II, 6-OHDA + Saline; Group III; Sham + Cystamine; Group IV, 6-OHDA + Cystamine. In total, 40 mice (n=10 per group) were utilized for each experiment, monitored daily for weight variation, and ultimately sacrificed by perfusion 24 hrs after the last cystamine (or vehicle) injection. The results are shown in **Figures 10,11** and **12.**

***Perfusion and tissue processing***

Animals were sacrificed under deep anesthesia with ketamine/xylazine (Vetalar, Bioniche, Belleville, ON/Rompun, Bayer, Toronto, ON). All mice were subjected to intracardiac perfusion with RNAse free 0.1 M phosphate-buffered saline (PBS). After intracardiac perfusion, brains were collected and the posterior brain of each mice, comprising the entire mesencephalon, was post-fixed in 4% PFA for additional immunohistochemistry and *in situ* hybridization analyses. The anterior brain, comprising the right and left striatum, was snap-frozen and used for HPLC and WB analyses.

**Catecholamine quantification by HPLC, TH immunohistochemistry** and **in situ hybridization for *Nurr1* and DAT** were conducted as described in Example 1.

***Densitometric measurements of Nurr1 and DAT mRNA levels***

Levels of autoradiographic labelling were quantified by computerized densitometry. Digitized brain images and their analyses were made with the same equipment as mentioned above. Optical density of the autoradiograms was translated in μCi/g of tissue using $^{14}C$ radioactivity standards (ARC 146-$^{14}C$ standards, American Radiolabelled Chemicals Inc., St. Louis, MO). *Nurr1* and DAT mRNA levels were measured in the substantia nigra compacta (SNc) using similar antero-posterior levels for all sections. The average labelling for each SNc level was calculated from 3 adjacent brain sections of the same mouse. Background intensities taken from white areas of the substantia nigra reticulata (SNr) devoid of *Nurr1* or DAT mRNA levels were subtracted from every measurement.

***Stereological quantification of TH-immunoreactive neurons***

The loss of DAergic neurons was determined by stereological counts of TH-immunoreactive cells (identifiable somas) under bright-field illumination. Every 10th section through the SNc was analyzed using Stereo investigator software (MicroBrightfield, Colchester, VT, USA) attached to an E800 Nikon microscope (Nikon Canada Inc., Mississauga, ON, Canada). After delineation of the SNc at low magnification (4X objective), a point grid was overlaid onto each section. For the most rostral level of SNc analyzed (bregma -3.08mm), the SNc was delineated by the visible boundaries with the medial terminal nucleus. For the intermediate (bregma -3.28mm) and most caudal levels of SNc analyzed (bregma -3.58mm), the structure was demarcated by the exit of the 3rd cranial nerve. Immunostained cells were counted by the optical fractionator method at higher magnification (20X objective). The counting variables were as follow: distance between counting frames (150 μm X 150 μm), counting frame size (75 μm) and guard zone thickness (1 μm). Cells were counted only if they did not intersect forbidden lines. The optical fractionator (Glaser and Glaser, 2000) method was used to count TH-positive (TH- and cresyl violet-positive) and TH-negative (cresyl violet-positive only) expressing cells. Stereological cell counts were performed blindly by two independent investigators. Note that the analyses of the TH-immunoreactive profiles were restricted to the SNc and thus excluded the ventral tegmental area (VTA).

***Apomorphine-induced rotation***

Rotational behavior is considered to represent a reliable physiological measure of DA depletion and asymmetric DA receptor stimulation. By applying DA agonists such as apomorphine (Anden et al., 1966; Ungerstedt et al., 1968), DA receptors are directly stimulated leading to rotation contralateral to the DA-depleted hemisphere. Mice were challenged with apomorphine (0.5 mg/kg, Sigma-Aldrich) at 3, 6, and 9 weeks after lesion, and rotational behaviors were assessed for 45 min using an automated rotometer system. Results were averaged and expressed as net rotation (= number of contralateral rotations - number of ipsilateral rotations) as previously described (Metz 2002). Animals were allowed to habituate for 5 min after the injection before the recording of rotations began.

***Stepping test***

The adjusting steps test was adapted from studies in rats (Lindner et al., 1995) and MPTP-treated mice (Blume et al., 2009). Mice were held by the base of the tail with their hindlimbs suspended above the table and moved backwards at a steady rate so that they traversed 1 meter distance over 3-4 s. The mice were video-recorded using a digital video camera (Sony Handcam, DCR-HC90E PAL) but the video was analyzed offline by counting the number of adjusting steps made with the contralateral or ipsilateral paw, relative to the lesioned hemisphere, over the total distance. Three trials were performed on mice at 3 weeks, 6 weeks and 9 weeks after surgery and median data were calculated across trials.

***Cylinder test***

The limb-use asymmetry (cylinder) test, which measures weight-bearing forelimb movements during rearing, has been shown to be a good indicator of nigrostriatal cell loss in unilateral 6-OHDA injected rats and mice (Schallert et al., 2000; Tillerson et al., 2001; Lundblad et al., 2004; Lancu et al., 2005). To examine side bias in spontaneous forelimb use during explorative activity, mice were placed individually inside a glass cylinder (10 cm diameter, 14 cm height), which was located in front of two vertical mirrors in order to be able to view the mice from all angles. Mice were immediately video recorded for 3 min. No habituation of the animals to the testing cylinder was allowed prior to video recording. Video recordings were then examined to count the number of supporting wall touches (contacts with fully extended digits) executed independently with the forelimb ipsilateral and contralateral to the lesion. A measure of forelimb use asymmetry was obtained by expressing the touches performed by the paw contralateral to the lesion (right paw) as a percentage of the total number of touches in each session.

***Statistical analyses***

All analyses are expressed as group mean $\pm$ S.E.M. Data were assessed by two-way ANOVA. When the two-way ANOVA yielded non-significant interaction terms the data were further analyzed for significance using the Tukey post-hoc multiple comparison test. In all cases, a P value of less than 0.05 was considered to be significant.

**REFERENCES:**

Allen P. Allen brain atlas. Seattle (WA): Allen Institute for Brain Science; 2008. Available from: http://www.brain-map.org.

Anden, N.E., Dahlstrom, A., Fuxe, K., Larsson, K., 1966. Functional role of the nigrostriatal dopamine neurons. Acta Pharmacol. Toxicol. 24,236-274.

Bacq Z. M. and Beaumariage M. L. (1965) Radioprotective action of cysteamine and cystamine in mice as a function of the period of time between injection of the protector and the start of X-Ray irradiation. Arch. Int. Pharmacodyn. Ther 153, 457-459.

Beaudry G, Langlois MC, Weppe I, Rouillard C, Levesque D. Contrasting (2000) patterns and cellular specificity of transcriptional regulation of the nuclear receptor nerve growth factor-inducible B by haloperidol and clozapine in the rat forebrain. J Neurochem 2000; 75:1694-702.

Benrabh H., Bourre J. M. and Lefauconnier J. M. (1995) Taurine transport at the blood-brain barrier: an in vivo brain perfusion study. Brain Res. 692, 57-65.

Bjorklund, A., Rosenblad, C., Winkler, C., Kirik, D., 1997. Studies on neuroprotective and regenerative effects of GDNF in a partial lesion model of Parkinson’s disease. Neurobiol Dis 4, 186-200.

Blume SR, Cass DK, Tseng KY. Stepping test in mice: a reliable approach in determining forelimb akinesia in MPTP-induced Parkinsonism. Exp Neurol 2009;219:208-11.

Borrell-Pages M., Canals J. M., Cordelieres F. P. et al. (2006) Cystamine and cysteamine increase brain levels of BDNF in Huntington disease via HSJ1b and transglutaminase. J. Clin. Invest 116, 1410-1424.

Bouckenooghe T., Remacle C. and Reusens B. (2006) Is taurine a functional nutrient? Curr: Opin. Clin. Nutr. Metab. Care 9, 728-733.

Bourasset F., Cisternino S., Temsamani J. and Scherrmann J. M. (2003) Evidence for an active transport of morphine-6-beta-d-glucuronide but not P-glycoprotein-mediated at the blood-brain barrier. J. Neurochem. 86, 1564-1567.

Bousquet M, Gibrat C, Saint-Pierre M, Julien C, Calon F, Cicchetti F. Modulation of brain-derived neurotrophic factor as a potential neuroprotective mechanism of action of omega-3 fatty acids in a parkinsonian animal model. Prog Neuropsychopharmacol Biol Psychiatry. 2009 Nov 13;33(8):1401-8. Epub 2009 Jul 24.

Bousquet M., Gibrat C.,Ouellet M., , Rouillard C., Calon F., and Cicchetti F. (2010) Cystaminemetabolism and brain transport properties: clinical implications for neurodegenerative diseases. J. Neurochem. (2010) 114, 1651-1658.

Bousquet M, Gue K, Emond V, Julien P, Kang JX, Cicchetti F, Calon F. Transgenic conversion of omega-6 into omega-3 fatty acids in a mouse model of Parkinson’s disease. J Lipid Res. (2011) Feb;52(2):263-71. Epub 2010 Nov 29.

Calon F., Morissette M., Goulet M., Grondin R., Blanchet P. J., Bedard P. J. and Di Paolo T. (1999) Chronic DI and D2 dopaminomimetic treatment of MPTP-denervated monkeys: effects on basal ganglia GABA(A)/benzodiazepine receptor complex and GABA content. Neurochem. Int 35, 81-91.

Calon F. et al., Effect of MPTP-induced denervation on basal ganglia GABA(B) receptors: correlation with dopamine concentrations and dopamine transporter. Synapse 2001, June 1;40(3):225-34.

Calon F. et al., Changes of GABA receptors and dopamine turnover in the postmortem brains of parkinsonians with levodopa-induced motor complications. Mov Disord. 2003 Mar;18(3):241-53.

Cavallini D., Scandurra R. and Demarco C. (1963) The enzymatic oxidation of cysteamine to hypotaurine in the presence of sulfide. J. Bio. Chem. 238, 2999-3005.

Coloso R. M., Hirschberger L. L., Dominy J. E., Lee J. I. and Stipanuk M. H (2006) Cysteamine dioxygenase: evidence for the physiological conversion of cysteamine to hypotaurine in rat and mouse tissues. Adv. Exp. Med. Biol. 583, 25-36.

Costantini LC, Cole D, Chaturvedi P, Isacson O. Immunophilin ligands can prevent progressivedopaminergic degeneration in animal models of Parkinson’s disease. Eur J Neurosci. Mar.

Cossette M, Parent A, Levesque D. Tyrosine hydroxylase-positive neurons intrinsic to the human striatum express the transcription factor Nurr1. Eur J NeuroSci. 2004;20:2089-95.

Dagenais C., Rousselle C., Pollack G. M. and Scherrmann J. M. (2000) Development of an in situ mouse brain perfusion model and its application to mdrla P-glycoprotein-deficient mice. J. Cereb. Blood Flow Metab. 20, 381-386.

Dedeoglu A., Kubilus J. K., Jeitner T. M. et al. (2002) Therapeutic effects of cystamine in a murine model of Huntington’s disease. J. Neurosci. 22, 8942-8950.

Dohil R., Fidler M., Gangoiti J. A., Kaskel F., Schneider J. A. and Barshop B. A. (2009) Twice-daily cysteamine bitartrate therapy for children with cystinosis. J. Pediatr. 156,71-75.

Dominy J., Eller S. and Dawson R. Jr (2004) Building biosynthetic schools: reviewing compartmentation of CNS taurine synthesis. Neurochem. Res. 29, 97-103.

Dominy J. E. Jr, Simmons C. R., Hirschberger L. L., Hwang J., Coloso R. M. and Stipanuk M. H. (2007) Discovery and characterization of a second mammalian thiol dioxygenase, cysteamine dioxygenase. J. Biol. Chem. 282, 25189-25198.

Dubinsky R. and Gray C. (2006) CYTE-I-HD: phase I dose finding and tolerability study of cysteamine (Cystagon) in Huntington’s disease. Mov. Disord. 21, 530-533.

Ewetz L. and Sorbo B. (1966) Characteristics of the cysteinesulfinate-forming enzyme system in rat liver. Biochim. Biophys. Acta 128, 296-305.

Fox J. H., Barber D. S., Singh B. et al. (2004) Cystamine increases L-cysteine levels in Huntington's disease transgenic mouse brain and in a PC12 model of polyglutamine aggregation. J. Neurochem. 91, 413-422.

French E. D., Vezzani A., Whetsell W. O. Jr. and Schwarcz R. (1986) Anti-excitotoxic actions of taurine in the rat hippocampus studied in vivo and in vitro. Adv. Exp. Med. Biol. 203, 349-362.

Gibrat C., Bousquet M., Saint-Pierre M., Levesque D., Calon F., Rouillard C. and Cicchetti F. (2010) Cystamine prevents MPTP-induced toxicity in young adult mice via the up-regulation of the brain-derived neurotrophic factor. Prog. Neuropsychopharmacol. Biol. Psychiatry 34, 193-203.

Gibrat C, Saint-Pierre M, Rouillard C, Cicchetti F. (2007). Neuroprotective properties of cystamine in acute vs chronic models of Parkinson's disease. The Canadian Journal of Neurological Sciences Suppl. 3 - S42.

Gibrat C, Bousquet M, Saint-Pierre M, Lévesque D, Calon F, Rouillard C, Cicchetti F. 2009. Neuroprotective mechanisms of cystamine in a murine model of Parkinson's disease. Parkinsonism and Related Disorders 15S2, S29-S199.

Gibrat C, Saint-Pierre M, Bousquet M, Levesque D, Rouillard C, Cicchetti F. Differences between subacute and chronic MPTP mice models: investigation of dopaminergic neuronal degeneration and alpha-synuclein inclusions. J Neurochem (2009 B);109:1469-82.

Green H. (1993) Human genetic diseases due to codon reiteration: relationship to an evolutionary mechanism. Cell 74, 955-956.

Huxtable R. J. (1992) Physiological actions of taurine. Physiol. Rev. 72, 101-163.

Iancu R, Mohapel P, Brundin P, Paul G. Behavioral characterization of a unilateral 6-OHDA-lesion model of Parkinson's disease in mice. Behav Brain Res 2005;162:1-10.

Jeitner T. M., Delikatny E. J., Ahlqvist J., Capper H. and Cooper A. J. (2005) Mechanism for the inhibition of transglutaminase 2 by cystamine. Biochem. Pharmacol. 69, 961-970.

Karpuj M. V., Becher M. W., Springer J. E., Chabas D., Youssef S., Pedotti R., Mitchell D. and Steinman L. (2002) Prolonged survival and decreased abnormal movements in transgenic model of Huntington disease, with administration of the transglutaminase inhibitor cystamine. Nat. Med. 8, 143-149.

Lapointe N, St-Hilaire M, Martinoli MG, Blanchet J, Gould P, Rouillard C, et (2004) Rotenone induces non-specific central nervous system and systemic toxicity. FASEB J. 2004;18:717-9.

Lee J. I., Londono M., Hirschberger L. L. and Stipanuk M. H. (2004) Regulation of cysteine dioxygenase and gamma-glutamylcysteine synthetase is associated with hepatic cysteine level. J Nutr. Biochem. 15, 112-122.

Lein ES, Hawrylycz MJ, et al. Genome-wide atlas of gene expression in the adult mouse brain. Nature 2007;445:168-76.

Leonardi R., Zhang Y. M., Rock C. O. and Jackowski S. (2005) Coenzyme A: back in action. Prog. Lipid Res. 44, 125-153.

Lindner MD, Winn SR, Baetge EE, Hammang JP, Gentile FT, Doherty E, et al. Implantation of encapsulated catecholamine and GDNF-producing cells in rats with unilateral dopamine depletions and parkinsonian symptoms. Exp Neurol 1995;132:62-76. 690.

Lundblad M, Picconi B, Lindgren H, Cenci MA. A model of I-DOPA-induced dyskinesia in 6-hydroxydopamine lesioned mice: relation to motor and cellular parameters of nigrostriatal function. Neurobiol Dis 2004;16:110-23.

Martignoni M., Groothuis G. M. and de Kanter R. (2006) Species differences between mouse, rat, dog, monkey and human CYP-mediated drag metabolism, inhibition and induction. Expert Opin. Drug Metab. Toxicol. 2, 875-894.

Muramatsu M., Kakita K., Nakagawa K. and Kuriyama K. (1978) A modulating role of taurine on release of acetylcholine and norepinephrine from neuronal tissues. Jpn. J. Pharmacol. 28, 259-268.

Oja S. S. and Saransaari P. (1996) Taurine as osmoregulator and neu-romodulator in the brain. Metab. Brain Dis. 11, 153-164.

Oldendorf W. H. and Szabo J. (1976) Amino acid assigmnent to one of three blood-brain barrier amino acid carriers. Am. J. Physiol. 230, 94-98.

Ouellet M., Emond V., Chen C. T., Julien C., Bourasset F., Oddo S., LaFerla E, Bazinet R. P. and Calon F. (2009) Diffusion of docosahexaenoic and eicosapentaenoic acids through the blood-brain barrier: and in situ cerebral perfusion study. Neurochem. Int. 55, 476-482.

Pasantes-Morales H., Arzate N. E. and Cruz C. (1981) The role of taurine in nervous tissue: its effects on ionic fluxes. Adv Exp Med Biol 139, 273-292.

Pinto J. T., Van Raamsdonk J. M., Leavitt B. R., Hayden M. R., Jeitner T. M., Thaler H. T., Krasnikov B. F. and Cooper A. J. (2005) Treatment of YAC128 mice and their wild-type littermates with cystamine does not lead to its accumulation in plasma or brain: implications for the treatment of Huntington disease. J Neurochem 94, 1087-1101.

Pinto J. T., Khomenko T., Szabo S., McLaren G. D., Denton T. T., Krasnikov B. F., Jeitner T. M. and Cooper A. J. (2009) Measurement of sulfur-containing compounds involved in the metabolism and transport of cysteamine and cystamine. Regional differences in cerebral metabolism. J Chromatogr B Analyt Technol Biomed Life Sci 877, 3434-3441.

Pitari G., Maurizi G., Flati V., Ursini C. L., Spera L., Dupre S. and Cavallini D. (1992) Enzymatic synthesis of S-aminoethyl-L-cysteine from pantetheine. Biochim Biophys Acta 1116, 27-33.

Saller C. F. and Czupryna M. J. (1989) gamma-Aminobutyric acid, glutamate, glycine and taurine analysis using reversed-phase high-performance liquid chromatography and ultraviolet detection of dansyl chloride derivatives. J Chromatogr 487, 167-172.

Santa T., Aoyama C., Fukushima T., Imai K. and Funatsu T. (2006) Suppression of thiol exchange reaction in the determination of reduced-form thiols by high-performance liquid chromatography with fluorescence detection after derivatization with fluorogenic benzofurazan reagent, 7-fluoro-2,1,3-benzoxadiazole-4-sulfonate and 4-aminosulfonyl-7-fluoro-2,1,3-benzoxadiazole. Biomed Chromatogr 20, 656-661.

Schallert T, Fleming SM, Leasure JL, Tillerson JL, Bland ST. CNS plasticity and assessment of forelimb sensorimotor outcome in unilateral rat models of stroke, cortical ablation, parkinsonism and spinal cord injury. Neuropharmacology 2000;39:777-87.

Sershen H. and Lajtha A. (1979) Inhibition pattern by analogs indicates the presence of ten or more transport systems for amino acids in brain cells. J Neurochem 32, 719-726.

Stack E. C., Ferro J. L., Kim J., Del Signore S. J., Goodrich S., Matson S., Hunt B. B., Cormier K., Smith K., Matson W. R., Ryu H. and Ferrante R. J. (2008) Therapeutic attenuation of mitochondrial dysfunction and oxidative stress in neurotoxin models of Parkinson’s disease. Biochim Biophys Acta 1782, 151-162.

Sun L., Xu S., Zhou M., Wang C., Wu Y. and Chan P. (2010) Effects of cysteamine on MPTP-induced dopaminergic neurodegeneration in mice. Brain Res. 1335, 74-82.

Tillerson JL, Cohen AD, Philhower J, Miller GW, Zigmond MJ, Schallert T. Forced limb-use effects on the behavioral and neurochemical effects of 6-hydroxydopamine. J Neurosci 2001;21:4427-35.

Tremblay M. E., Saint-Pierre M., Bourhis E., Levesque D., Rouillard C. and Cicchetti F. (2006) Neuroprotective

effects of cystamine in aged parkinsonian mice. Neurobiol Aging 27, 862-870.

Ungerstedt, U., 1968. 6-Hydroxydopamine-induced degeneration of central monoamine neurons. Eur. J. Pharmacol. 5, 5107-5110.

Wade L. A. and Katzman R. (1975) Synthetic amino acids and the nature of L-DOPA transport at the blood-brain barrier. J Neurochem 25, 837-842.

Wade L. A. and Brady H. M. (1981) Cysteine and cystine transport at the blood-brain barrier. J Neurochem 37, 730-734.

Wang X., Sarkar A., Cicchetti F., Yu M., Zhu A., Jokivarsi K., Saint-Pierre M. and Brownell A. L. (2005) Cerebral PET imaging and histological evidence of transglutaminase inhibitor cystamine induced neuroprotection in transgenic R6/2 mouse model of Huntington's disease. J Neurol Sci 231, 57-66.

Zetterstrom, Rolf H. Ludmila Solomin, Lottie Jansson,Barry J. Hoffer, Lars Olson, Thomas Perlmann. Dopamine neuron agenesis in Nurr1-deficient mice. Science 1997 Apr 11;276(5310):248-50.

## Claims

**1.** A therapeutically effective amount of at least one cystamine analogue or a pharmaceutically acceptable salt thereof, for use as a neurorestorative agent for the treatment of a Parkinson's disease patient, wherein the patient is:

a Stage II patient according to Hoehn and Yahr rating;

- a Stage III patient according to Hoehn and Yahr rating; or
- a Stage IV patient according to Hoehn and Yahr rating;

and wherein the cystamine analogue is cysteamine, cystamine, taurine or hypotaurine or a pharmaceutically acceptable salt thereof.

**2.** The therapeutically effective amount of at least one cystamine analogue or a pharmaceutical acceptable salt thereof for use according to claim 1, wherein the patient is:

- a Stage II or III patient according to Hoehn and Yahr rating.

**3.** The therapeutically effective amount of at least one cystamine analogue or a pharmaceutically acceptable salt thereof for use according to claim 1, wherein the patient is a Stage III patient according to Hoehn and Yahr rating.

**4.** The therapeutically effective amount of at least one cystamine analogue or a pharmaceutically acceptable salt thereof for use according to any one of claims 1 to 3, wherein the therapeutically effective amount of at least one cystamine analogue or a pharmaceutically acceptable salt thereof is:

a) in the range of about 0.1 to about 750 mg/kg of body weight per day;
b) in the range of about 0.5 to about 60 mg/kg/day; or
c) in the range of about 1 to about 20 mg/kg/day.

**5.** The therapeutically effective amount of at least one cystamine analogue or a pharmaceutically acceptable salt thereof for use according to any one of claim 1 to 3, wherein the therapeutically effective amount of at least one cystamine analogue or a pharmaceutically acceptable salt thereof is suited to be administered in unit dosage form containing:

a) 5 to 2000 mg of active ingredient per unit dosage form;
b) 10 to 1500 mg of active ingredient per unit dosage form;
c) 20 to 1000 mg of active ingredient per unit dosage form; or
d) 50 to 700 mg of active ingredient per unit dosage form.

**6.** A pharmaceutical composition comprising at least one cystamine analogue or pharmaceutically acceptable salts thereof as defined in any one of claims 1 to 5 for use as a neurorestoration agent for the treatment of a Parkinson's disease patient, wherein the patient is a Stage II patient according to Hoehn and Yahr rating; a Stage III patient according to Hoehn and Yahr rating; or a Stage IV patient according to Hoehn and Yahr rating.

**7.** A combination comprising at least one cystamine analogue as defined in one of claims 1 to 5 and cysteine or pharmaceutically acceptable salts thereof for use as a neurorestorative agent for the treatment of a Parkinson's disease patient, wherein the patient is a Stage II patient according to Hoehn and Yahr rating; a Stage III patient according to Hoehn and Yahr rating; or a Stage IV patient according to Hoehn and Yahr rating.

**8.** The combination for use according to claim 7, wherein the cystamine analogue and cysteine are present in a ratio of:

a) 10:1 to 1:10 of cystamine analogue and cysteine respectively; or
b) 1:1 of cystamine analogue and cysteine respectively.

**9.** A pharmaceutical composition comprising at least one cystamine analogue or pharmaceutically acceptable salts thereof as defined in any one of claims 1 to 5 and comprising cysteine or pharmaceutically acceptable salts thereof for use as a neurorestorative agent for the treatment of a Parkinson's disease patient, wherein the patient a Stage II patient according to Hoehn and Yahr; a Stage III patient according to Hoehn and Yahr rating; or a Stage IV patient according to Hoehn and Yahr rating.

**10.** The composition for use according to claim 9, wherein the cystamine analogue and cysteine are present in a ratio of:

a) 10:1 to 1:10 of cystamine analogue and cysteine respectively or
b) 1:1 of cystamine analogue and cysteine respectively.

**11.** The combination for use according to claim 7 or 8 or the composition of claim 9 or 10, wherein the cystamine analogue and cysteine are suited to be used sequentially.

**12.** The combination for use according to claim 7 or 8 or the composition of claim 9 or 10, wherein the cystamine analogue and cysteine are suited to be used simultaneously.

**13.** The therapeutically effective amount of at least one cystamine analogue or a pharmaceutical acceptable salt thereof for use according to any one of claims 1 to 5, the composition according to claim 6, 9 or 10, or the combination for use according claim 7 or 8, wherein the cystamine analogue is cystamine or cysteamine or a pharmaceutically acceptable salt thereof.

**14.** The therapeutically effective amount of at least one cystamine analogue or a pharmaceutically acceptable salt thereof for use according to any one of claims 1 to 5, the composition according to claim 6, 9 or 10, or the combination for use according claim 7 or 8, wherein the cystamine analogue is cystamine or a pharmaceutically acceptable salt thereof.

**15.** The therapeutically effective amount of at least one cystamine analogue or a pharmaceutical acceptable salt thereof for use according to any one of claims 1 to 5, the composition according to claim 6, 9 or 10, or the combination for use according claim 7 or 8, wherein the cystamine analogue is cysteamine or a pharmaceutical acceptable salt thereof.

## Patentansprüche

**1.** Therapeutisch wirksame Menge von mindestens einem Cystaminanalogon oder einem pharmazeutisch verträglichen Salz davon zur Verwendung als Neurorestorationsmittel zur Behandlung eines Parkinson-Patienten, wobei der Patient Folgendes ist:

- ein Stadium-II-Patient nach Hoehn- und Yahr-Einstufung;
- ein Stadium-III-Patient nach Hoehn- und Yahr-Einstufung;
- ein Stadium-IV-Patient nach Hoehn- und Yahr-Einstufung;

und wobei das Cystaminanalogon Cysteamin, Cystamin, Taurin oder Hypotaurin oder ein pharmazeutisch verträgliches Salz davon ist.

2. Therapeutisch wirksame Menge von mindestens einem Cystaminanalogon oder einem pharmazeutisch verträglichen Salz davon zur Verwendung nach Anspruch 1, wobei der Patient Folgendes ist:

 - ein Stadium-II oder -III-Patient nach Hoehn- und Yahr-Einstufung.

3. Therapeutisch wirksame Menge von mindestens einem Cystaminanalogon oder einem pharmazeutisch verträglichen Salz davon zur Verwendung nach Anspruch 1, wobei der Patient ein Stadium-III-Patient nach Hoehn- und Yahr-Einstufung ist.

4. Therapeutisch wirksame Menge von mindestens einem Cystaminanalogon oder eines pharmazeutisch verträglichen Salzes davon zur Verwendung nach einem der Ansprüche 1 bis 3, wobei die therapeutisch wirksame Menge mindestens eines Cystaminanalogons oder eines pharmazeutisch verträglichen Salzes davon wie folgt ist:

 a) im Bereich von etwa 0,1 bis etwa 750 mg/kg Körpergewicht pro Tag;
 b) im Bereich von etwa 0,5 bis etwa 60 mg/kg/Tag; oder
 c) im Bereich von etwa 1 bis etwa 20 mg/kg/Tag.

5. Therapeutisch wirksame Menge von mindestens einem Cystaminanalogon oder eines pharmazeutisch verträglichen Salzes davon zur Verwendung nach einem der Ansprüche 1 bis 3, wobei die therapeutisch wirksame Menge mindestens eines Cystaminanalogons oder eines pharmazeutisch verträglichen Salzes davon geeignet ist, um in einer Einheitsdosierungsform verabreicht zu werden, die enthält:

 a) 5 bis 2000 mg Wirkstoff pro Einheitsdosierungsform;
 b) 10 bis 1500 mg Wirkstoff pro Einheitsdosierungsform;
 c) 20 bis 1000 mg Wirkstoff pro Einheitsdosierungsform; oder
 d) 50 bis 700 mg Wirkstoff pro Einheitsdosierungsform.

6. Pharmazeutische Zusammensetzung, umfassend mindestens ein Cystaminanalogon oder pharmazeutisch verträgliche Salze davon wie in einem der Ansprüche 1 bis 5 definiert, zur Verwendung als Neurorestorationsmittel zur Behandlung eines Parkinson-Patienten, wobei der Patient ein Stadium-II-Patient nach Hoehn- und Yahr-Einstufung; ein Stadium-III-Patient nach Hoehn- und Yahr-Einstufung; oder ein Stadium-IV-Patient nach Hoehn- und Yahr-Einstufung ist.

7. Kombination, umfassend mindestens ein Cystaminanalogon wie in einem der Ansprüche 1 bis 5 definiert, und Cystein oder pharmazeutisch verträgliche Salze davon zur Verwendung als Neurorestorationsmittel zur Behandlung eines Parkinson-Patienten, wobei der Patient ein Stadium-II-Patient nach Hoehn- und Yahr-Einstufung; ein Stadium-III-Patient nach Hoehn- und Yahr-Einstufung; oder ein Stadium-IV-Patient nach Hoehn- und Yahr-Einstufung ist.

8. Kombination zur Verwendung nach Anspruch 7, wobei das Cystaminanalogon und Cystein in den folgenden Verhältnissen vorliegen:

 a) 10:1 bis 1:10 zwischen Cystaminanalogon bzw. Cystein; oder
 b) 1:1 zwischen Cystaminanalogon bzw. Cystein.

9. Pharmazeutische Zusammensetzung, umfassend mindestens ein Cystaminanalogon oder pharmazeutisch verträgliche Salze davon wie in einem der Ansprüche 1 bis 5 definiert, und umfassend Cystein oder pharmazeutisch verträgliche Salze davon zur Verwendung als Neurorestorationsmittel zur Behandlung eines Parkinson-Patienten, wobei der Patient ein Stadium-II-Patient nach Hoehn- und Yahr-Einstufung; ein Stadium-III-Patient nach Hoehn- und Yahr-Einstufung; oder ein Stadium-IV-Patient nach Hoehn- und Yahr-Einstufung ist.

10. Zusammensetzung zur Verwendung nach Anspruch 9, wobei das Cystaminanalogon und Cystein in den folgenden Verhältnissen vorhanden sind:

 a) 10:1 bis 1:10 zwischen Cystaminanalogon bzw. Cystein; oder
 b) 1:1 zwischen Cystaminanalogon bzw. Cystein.

11. Kombination zur Verwendung nach Anspruch 7 oder 8 oder Zusammensetzung nach Anspruch 9 oder 10, wobei das Cystaminanalogon und Cystein zur Verwendung nacheinander geeignet sind.

12. Kombination zur Verwendung nach Anspruch 7 oder 8 oder Zusammensetzung nach Anspruch 9 oder 10, wobei das Cystaminanalogon und Cystein zur gleichzeitigen Verwendung geeignet sind.

13. Therapeutisch wirksame Menge von mindestens einem Cystaminanalogon oder eines pharmazeutisch verträglichen Salzes davon zur Verwendung nach einem der Ansprüche 1 bis 5, Zusammensetzung nach Anspruch 6, 9 oder 10 oder Kombination zur Verwendung nach Anspruch 7 oder 8, wobei das Cystaminanalogon Cystamin oder Cysteamin oder ein pharmazeutisch verträgliches Salz davon ist.

14. Therapeutisch wirksame Menge von mindestens einem Cystaminanalogon oder eines pharmazeutisch verträglichen Salzes davon zur Verwendung nach einem der Ansprüche 1 bis 5, Zusammensetzung nach Anspruch 6, 9 oder 10 oder Kombination zur Verwendung nach Anspruch 7 oder 8, wobei das Cystaminanalogon Cystamin oder ein pharmazeutisch verträgliches Salz davon ist.

15. Therapeutisch wirksame Menge von mindestens einem Cystaminanalogon oder eines pharmazeutisch verträglichen Salzes davon zur Verwendung nach einem der Ansprüche 1 bis 5, Zusammensetzung nach Anspruch 6, 9 oder 10 oder Kombination zur Verwendung nach Anspruch 7 oder 8, wobei das Cystaminanalogon Cysteamin oder ein pharmazeutisch verträgliches Salz davon ist.

**Revendications**

1. Quantité thérapeutiquement efficace d'au moins un analogue de la cystamine ou de l'un de ses sels pharmaceutiquement acceptables, pour une utilisation en tant qu'agent neurorestaurateur pour le traitement d'un patient atteint de la maladie de Parkinson, où le patient est :

   un patient au stade II selon la classification de Hoehn et Yahr ;
   un patient au stade III selon la classification de Hoehn et Yahr ; ou
   un patient au stade IV selon la classification de Hoehn et Yahr ;
   et où l'analogue de la cystamine est la cystéamine, la cystamine, la taurine ou l'hypotaurine ou l'un de leurs sels pharmaceutiquement acceptables.

2. Quantité thérapeutiquement efficace d'au moins un analogue de la cystamine ou de l'un de ses sels pharmaceutiquement acceptables pour une utilisation selon la revendication 1, où le patient est :

   un patient au stade II ou III selon la classification de Hoehn et Yahr.

3. Quantité thérapeutiquement efficace d'au moins un analogue de la cystamine ou de l'un de ses sels pharmaceutiquement acceptables pour une utilisation selon la revendication 1, où le patient est un patient au stade III selon la classification de Hoehn et Yahr.

4. Quantité thérapeutiquement efficace d'au moins un analogue de la cystamine ou de l'un de ses sels pharmaceutiquement acceptables pour une utilisation selon l'une quelconque des revendications 1 à 3, où la quantité thérapeutiquement efficace d'au moins un analogue de la cystamine ou de l'un de ses sels pharmaceutiquement acceptables se situe :

   a) dans la gamme d'environ 0,1 à environ 750 mg/kg de poids corporel par jour ;
   b) dans la gamme d'environ 0,5 à environ 60 mg/kg/jour ; ou
   c) dans la gamme d'environ 1 à environ 20 mg/kg/jour.

5. Quantité thérapeutiquement efficace d'au moins un analogue de la cystamine ou de l'un de ses sels pharmaceutiquement acceptables pour une utilisation selon l'une quelconque des revendications 1 à 3, où la quantité thérapeutiquement efficace d'au moins un analogue de la cystamine ou de l'un de ses sels pharmaceutiquement acceptables est adaptée pour être administrée sous une forme galénique unitaire contenant :

   a) 5 à 2000 mg de principe actif par forme galénique unitaire ;

b) 10 à 1500 mg de principe actif par forme galénique unitaire ;
c) 20 à 1000 mg de principe actif par forme galénique unitaire ; ou
d) 50 à 700 mg de principe actif par forme galénique unitaire.

**6.** Composition pharmaceutique comprenant au moins un analogue de la cystamine ou de l'un de ses sels pharmaceutiquement acceptables tel que défini dans l'une quelconque des revendications 1 à 5 pour une utilisation en tant qu'agent de neurorestauration pour le traitement d'un patient atteint de la maladie de Parkinson, où le patient est un patient au stade II selon la classification de Hoehn et Yahr ; un patient au stade III selon la classification de Hoehn et Yahr ; ou un patient au stade IV selon la classification de Hoehn et Yahr.

**7.** Combinaison comprenant au moins un analogue de la cystamine tel que défini dans l'une quelconque des revendications 1 à 5 et de la cystéine ou l'un de ses sels pharmaceutiquement acceptables pour une utilisation en tant qu'agent neurorestaurateur pour le traitement d'un patient atteint de la maladie de Parkinson, où le patient est un patient au stade II selon la classification de Hoehn et Yahr ; un patient au stade III selon la classification de Hoehn et Yahr ; ou un patient au stade IV selon la classification de Hoehn et Yahr.

**8.** Combinaison pour une utilisation selon la revendication 7, dans laquelle l'analogue de la cystamine et la cystéine sont présents dans un rapport de :

a) 10:1 à 1:10 d'analogue de la cystamine et de cystéine respectivement ; ou
b) 1:1 d'analogue de la cystamine et de cystéine respectivement.

**9.** Composition pharmaceutique comprenant au moins un analogue de la cystamine ou l'un de ses sels pharmaceutiquement acceptables tel que défini dans l'une quelconque des revendications 1 à 5 et comprenant de la cystéine ou ses sels pharmaceutiquement acceptables pour une utilisation en tant qu'agent neurorestaurateur pour le traitement d'un patient atteint de la maladie de Parkinson, où le patient est un patient au stade II selon la classification de Hoehn et Yahr ; un patient au stade III selon la classification de Hoehn et Yahr ; ou un patient au stade IV selon la classification de Hoehn et Yahr.

**10.** Composition pour une utilisation selon la revendication 9, dans laquelle l'analogue de la cystamine et la cystéine sont présents dans un rapport de :

a) 10:1 à 1:10 d'analogue de la cystamine et de cystéine respectivement ou
b) 1:1 d'analogue de la cystamine et de cystéine respectivement.

**11.** Combinaison pour une utilisation selon la revendication 7 ou 8 ou composition selon la revendication 9 ou 10, dans laquelle l'analogue de la cystamine et la cystéine sont adaptés pour être utilisées séquentiellement.

**12.** Combinaison pour une utilisation selon la revendication 7 ou 8 ou composition selon la revendication 9 ou 10, dans laquelle l'analogue de la cystamine et la cystéine sont adaptés pour être utilisées simultanément.

**13.** Quantité thérapeutiquement efficace d'au moins un analogue de la cystamine ou de l'un de ses sels pharmaceutiquement acceptables pour une utilisation selon l'une quelconque des revendications 1 à 5, composition selon la revendication 6, 9 ou 10, ou combinaison pour une utilisation selon la revendication 7 ou 8, dans laquelle l'analogue de la cystamine est la cystamine ou la cystéamine ou l'un de leurs sels pharmaceutiquement acceptables.

**14.** Quantité thérapeutiquement efficace d'au moins un analogue de la cystamine ou de l'un de ses sels pharmaceutiquement acceptables pour une utilisation selon l'une quelconque des revendications 1 à 5, composition selon la revendication 6, 9 ou 10, ou combinaison pour une utilisation selon la revendication 7 ou 8, dans laquelle l'analogue de la cystamine est la cystamine ou l'un de ses sels pharmaceutiquement acceptables.

**15.** Quantité thérapeutiquement efficace d'au moins un analogue de la cystamine ou de l'un de ses sels pharmaceutiquement acceptables pour une utilisation selon l'une quelconque des revendications 1 à 5, composition selon la revendication 6, 9 ou 10, ou combinaison pour une utilisation selon la revendication 7 ou 8, dans laquelle l'analogue de la cystamine est la cystéamine ou l'un de ses sels pharmaceutiquement acceptables.

# Figure 1

a) b)

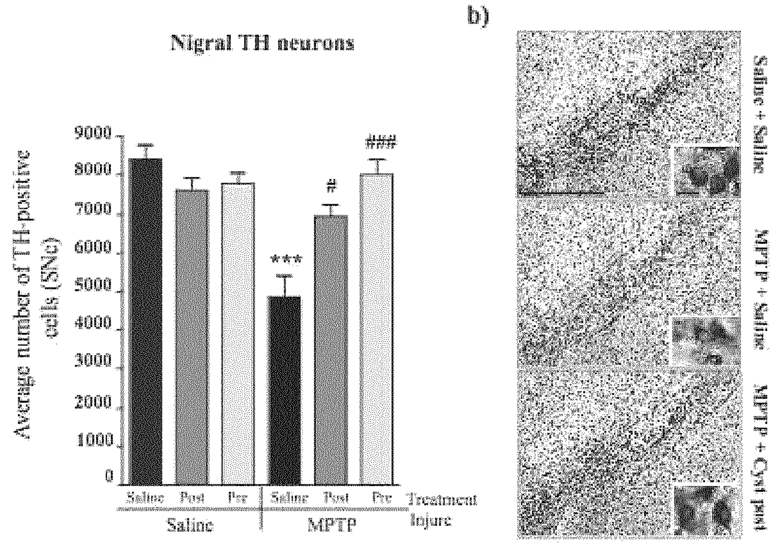


c)

| Treatments | Stereological cell count | | |
|---|---|---|---|
| | Cresyl Violet+ | TH+ | TH+ & Cresyl+ |
| Saline + Saline | 424 ± 77 | 8414 ± 354 | 8838 ± 374 |
| Saline + Cyst post-treatment | 360 ± 96 | 7605 ± 320 | 7965 ± 329 |
| Saline + Cyst pre-treatment | 521 ± 85 | 7804 ± 263 | 8325 ± 269 |
| MPTP + Saline | 653 ± 167 | 4846 ± 545 *** | 5499 ± 465 *** |
| MPTP + Cyst post-treatment | 512 ± 81 | 6925 ± 309 # | 7437 ± 329 ## |
| MPTP + Cyst pre-treatment | 544 ± 104 | 8037 ± 388 ### | 8581 ± 425 ### |

*PRE-TREATMENT SCHEDULE*

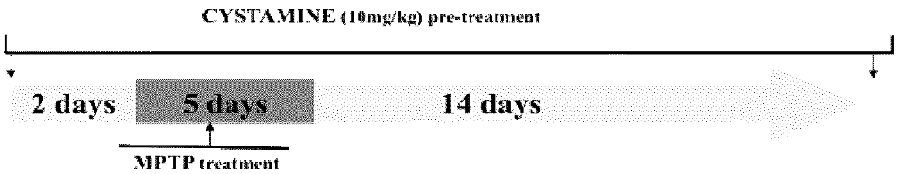


*POST-TREATMENT SCHEDULE*

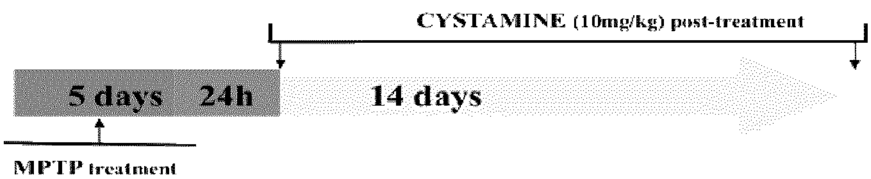

# Figure 2

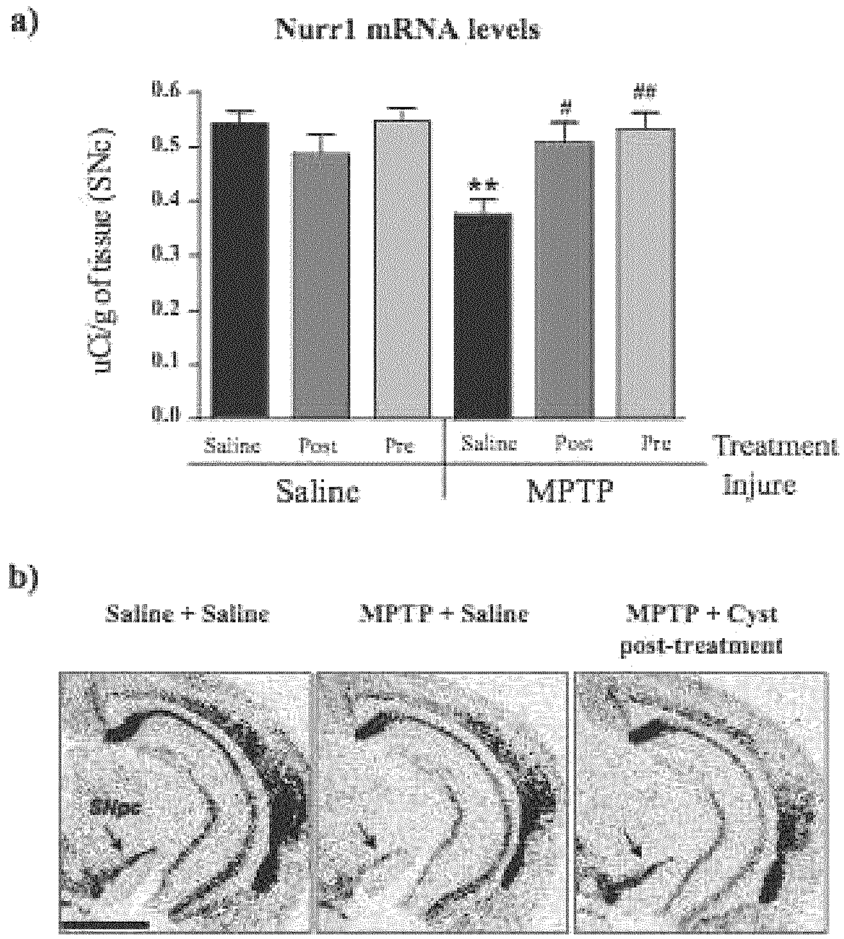
a)
Nurr1 mRNA levels
uCi/g of tissue (SNc)
0.0
0.1
0.2
0.3
0.4
0.5
0.6
**
#
##
Saline
Post
Pre
Saline
Post
Pre
Treatment
Saline
MPTP
Injure
b)
Saline + Saline
MPTP + Saline
MPTP + Cyst
post-treatment
SNpc

# Figure 3

a)

b)

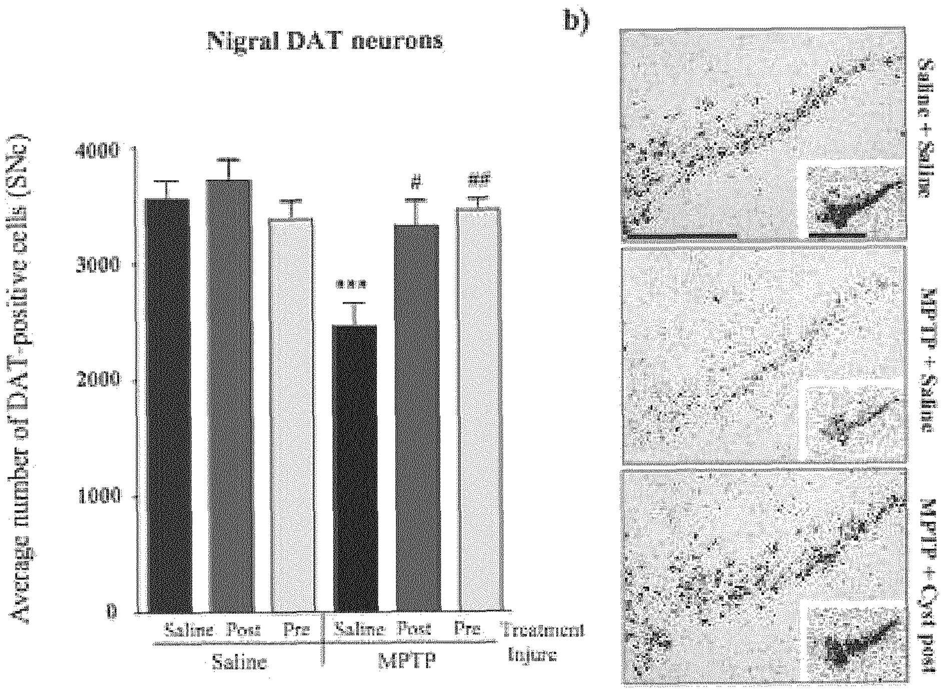


c)

| Treatments | Stereological cell count | optical density |
|---|---|---|
| Saline + Saline | 3571 ± 152 | 2.644 ± 0.1145 |
| Saline + Cyst post-treatment | 3724 ± 183 | 2.554 ± 0.1604 |
| Saline + Cyst pre-treatment | 3383 ± 153 | 2.453 ± 0.1522 |
| MPTP + Saline | 2458 ± 210 *** | 1.974 ± 0.1342 * |
| MPTP + Cyst post-treatment | 3325 ± 227 # | 2.474 ± 0.1534 |
| MPTP + Cyst pre-treatment | 3457 ± 112 ## | 2.748 ± 0.1868 ## |

# Figure 4

a)

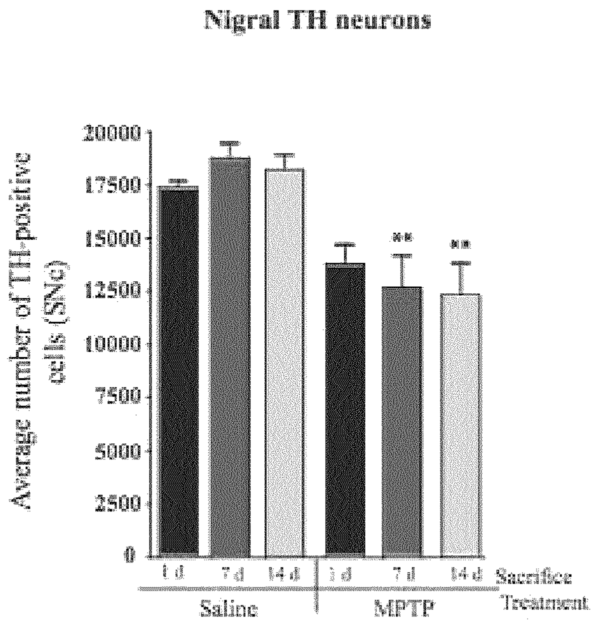


b)

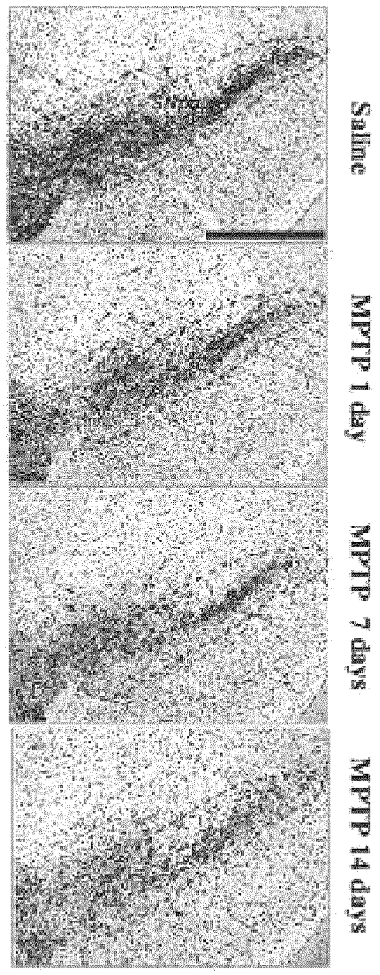


c)

| Treatments | Stereological cell count | | |
|---|---|---|---|
| | Cresyl Violet+ | TH+ | TH+ & Cresyl+ |
| Saline 1 d | 600 ± 285 | 17410 ± 287 | 18010 ± 80 |
| Saline 7 d | 988 ± 249 | 18800 ± 694 | 19788 ± 849 |
| Saline 14 d | 1912 ± 274 | 18290 ± 741 | 20202± 562 |
| MPTP 1 d | 613 ± 186 | 13830 ± 885 | 14443± 1036 |
| MPTP 7 d | 1651± 863 | 12830 ± 1449 ** | 14481± 1159** |
| MPTP 14 d | 2523 ± 663 | 12320 ± 1537 ** | 14843± 1305** |

# Figure 5

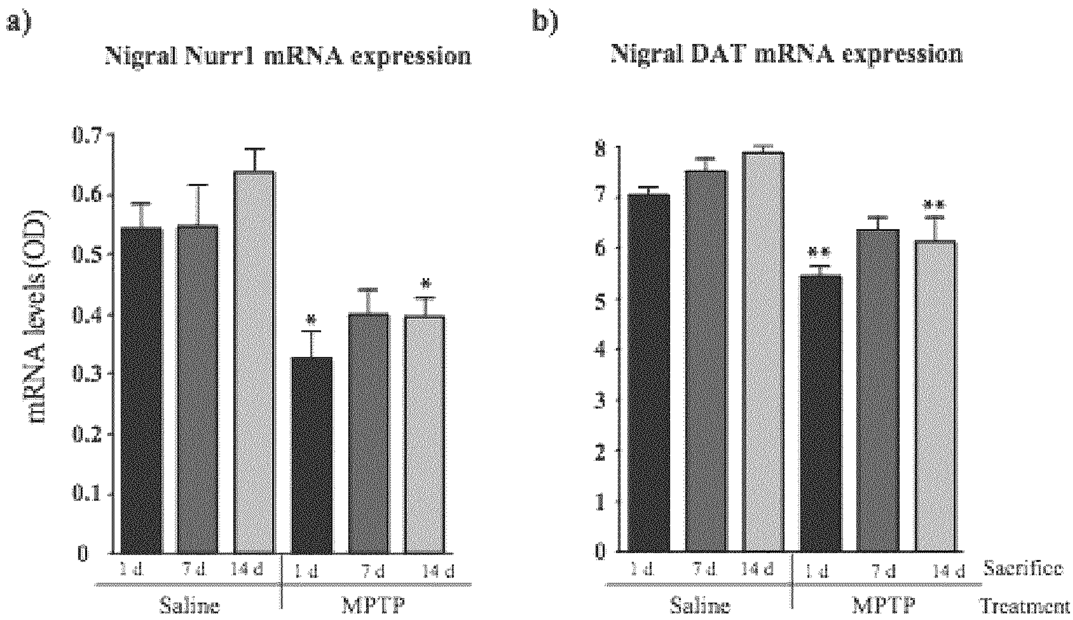


# Figure 6

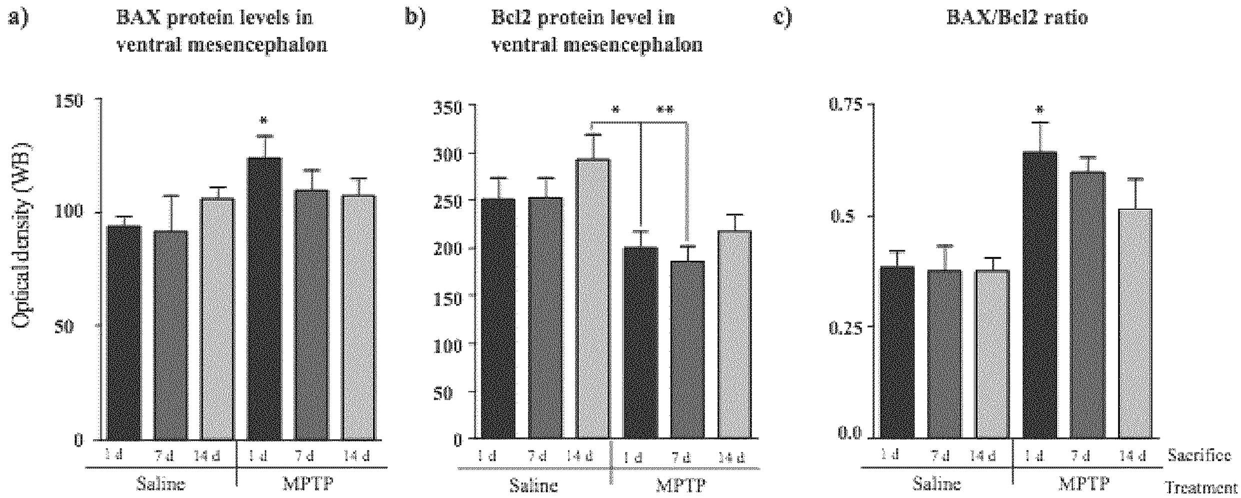

**Figure 7**

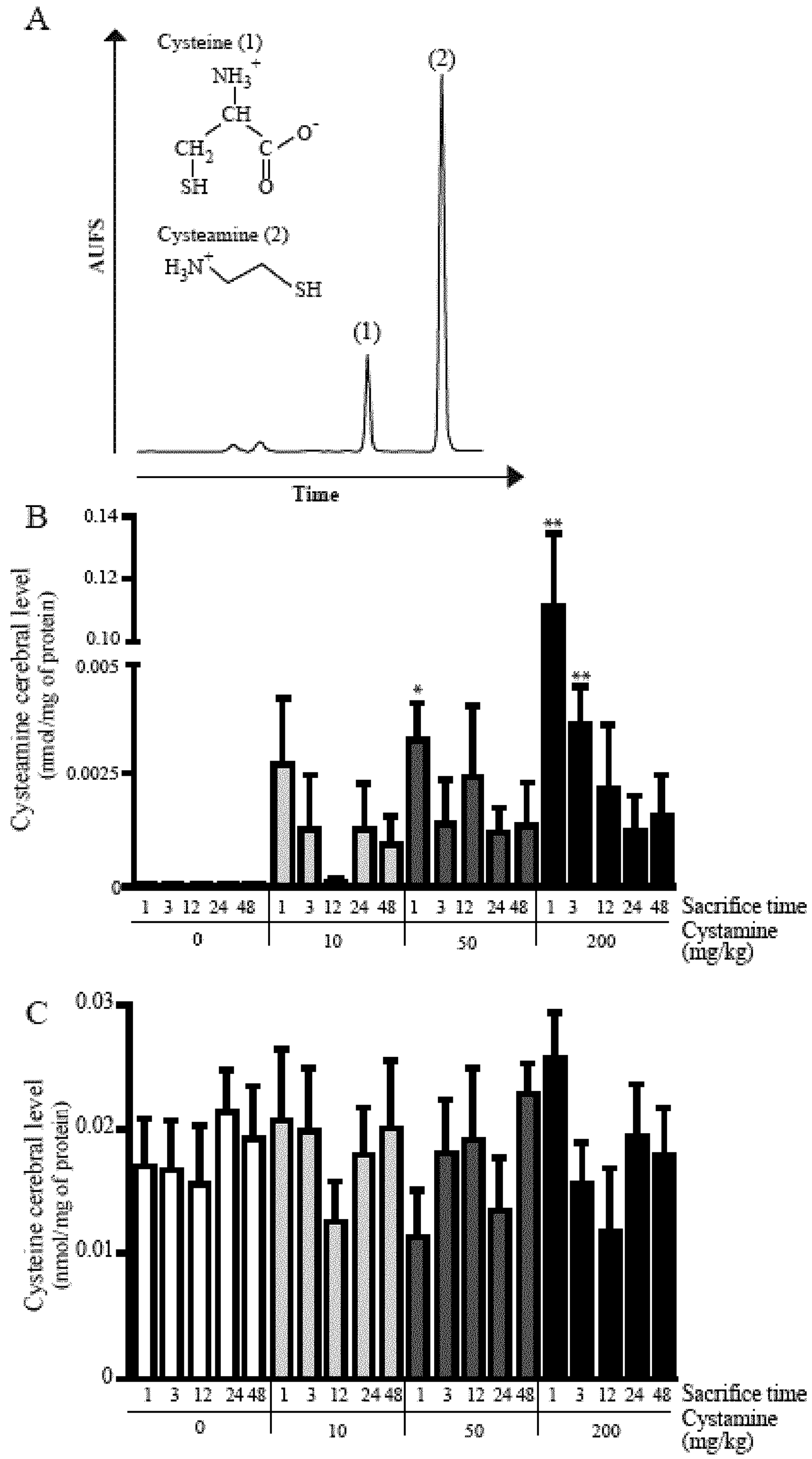
A
Cysteine (1)
Cysteamine (2)
AUFS
(1)
(2)
Time
B
Cysteamine cerebral level (nmol/mg of protein)
0.14
0.12
0.10
0.005
0.0025
0
*
**
**
1 3 12 24 48 1 3 12 24 48 1 3 12 24 48 1 3 12 24 48 Sacrifice time
0 10 50 200 Cystamine (mg/kg)
C
Cysteine cerebral level (nmol/mg of protein)
0.03
0.02
0.01
0
1 3 12 24 48 1 3 12 24 48 1 3 12 24 48 1 3 12 24 48 Sacrifice time
0 10 50 200 Cystamine (mg/kg)

**Figure 8**

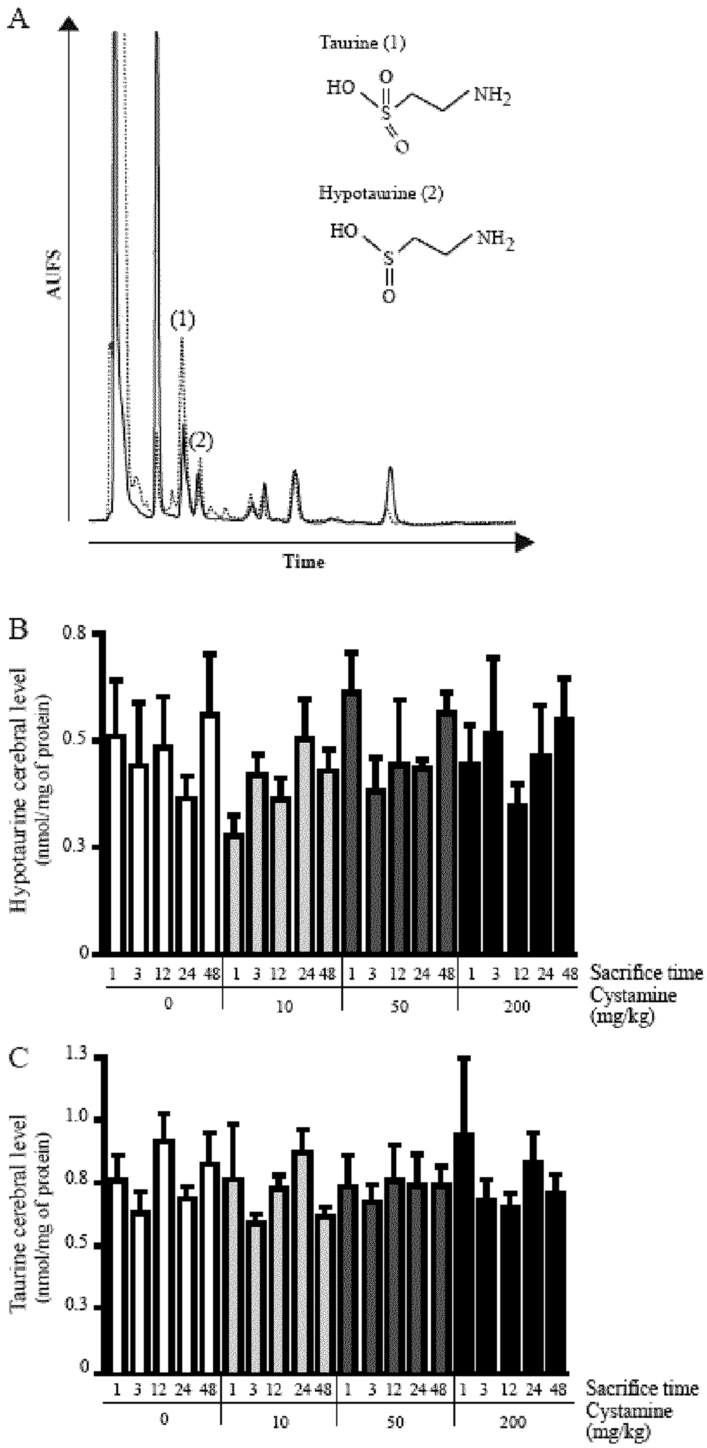
A
AUFS
Time
(1)
(2)
Taurine (1)
Hypotaurine (2)
B
Hypotaurine cerebral level (nmol/mg of protein)
0.8
0.5
0.3
0
1 3 12 24 48 1 3 12 24 48 1 3 12 24 48 1 3 12 24 48
Sacrifice time
0 10 50 200
Cystamine (mg/kg)
C
Taurine cerebral level (nmol/mg of protein)
1.3
1.0
0.8
0.5
0.3
0
1 3 12 24 48 1 3 12 24 48 1 3 12 24 48 1 3 12 24 48
Sacrifice time
0 10 50 200
Cystamine (mg/kg)

Figure 9

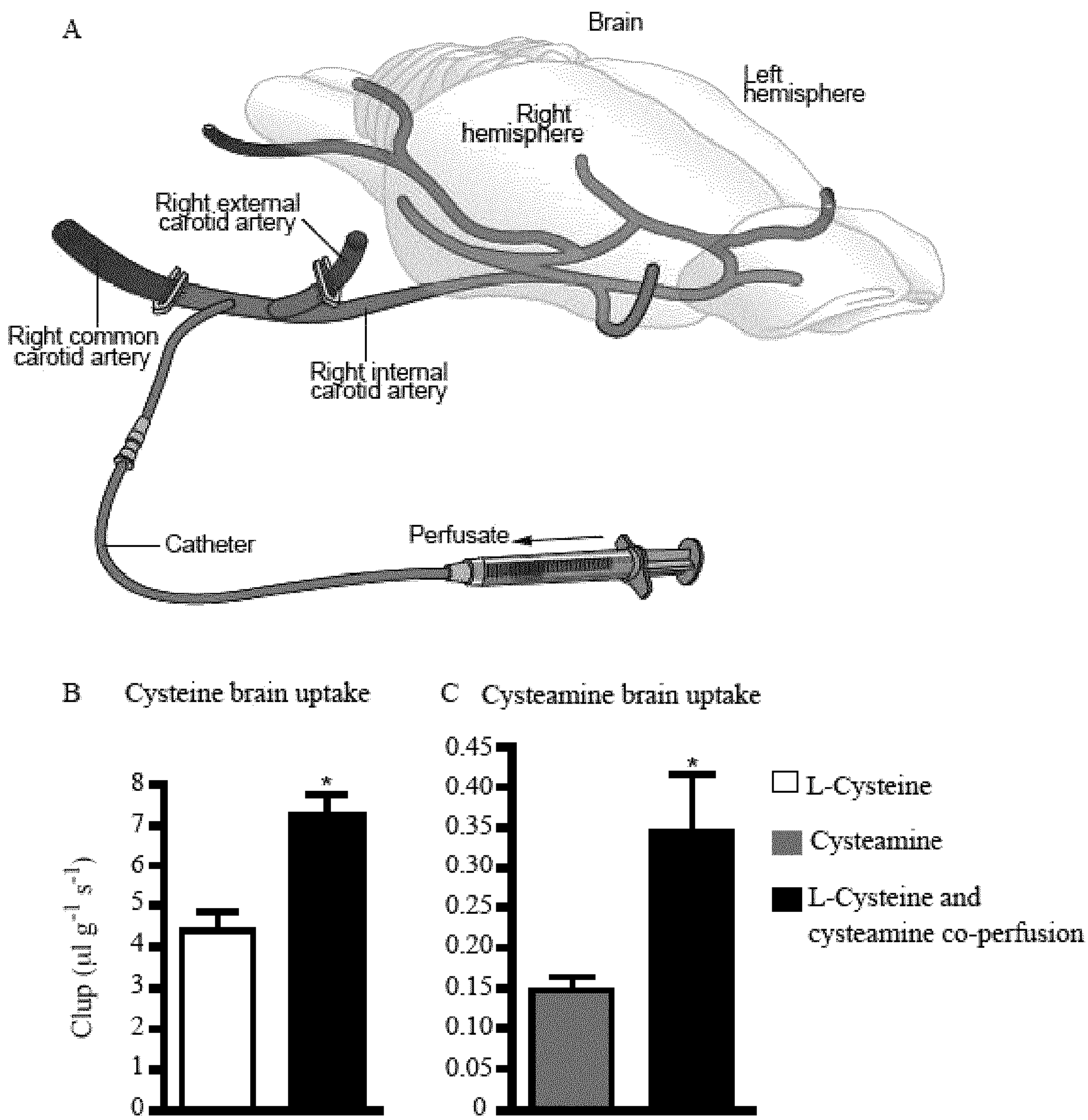
A
Brain
Left hemisphere
Right hemisphere
Right external carotid artery
Right common carotid artery
Right internal carotid artery
Catheter
Perfusate
B Cysteine brain uptake
C Cysteamine brain uptake
Clup (µl g-1 s-1)
0
1
2
3
4
5
6
7
8
*
0
0.05
0.10
0.15
0.20
0.25
0.30
0.35
0.40
0.45
*
L-Cysteine
Cysteamine
L-Cysteine and cysteamine co-perfusion

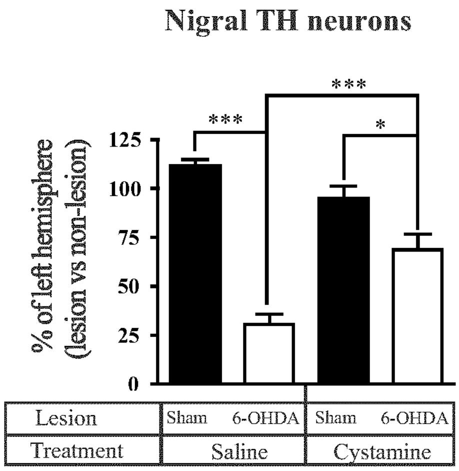


Figure 10a

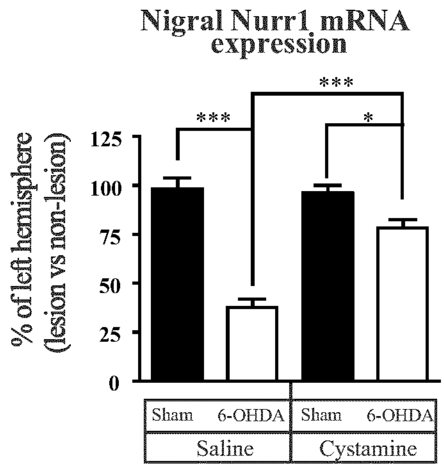


Figure 10b

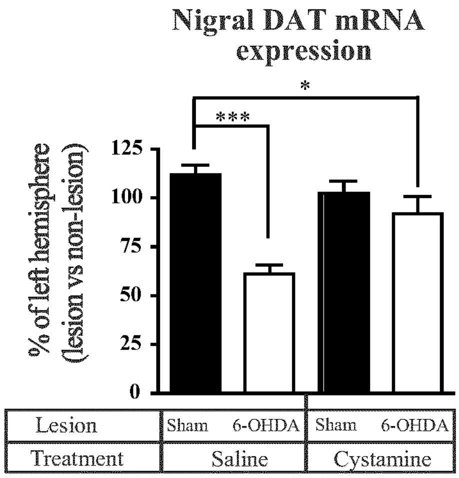


Figure 10c

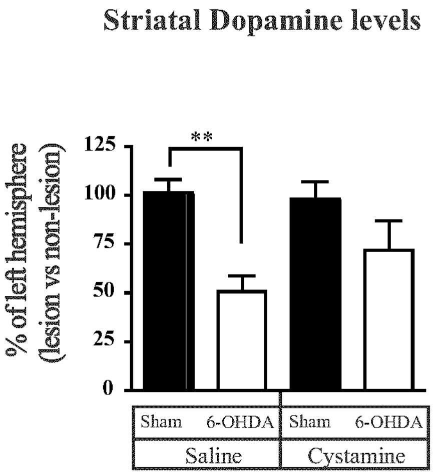


Figure 10d

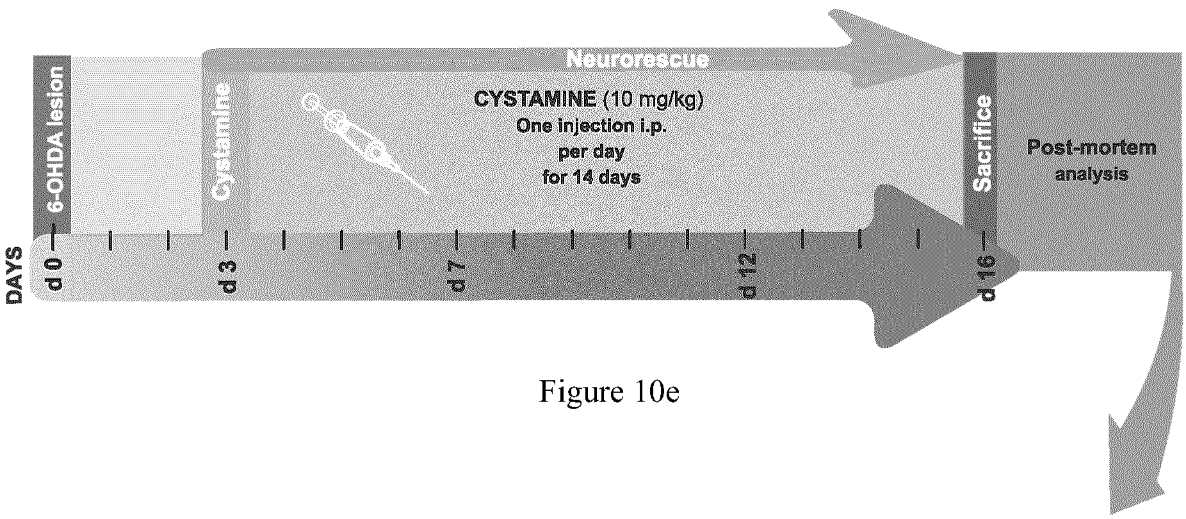


Figure 10e

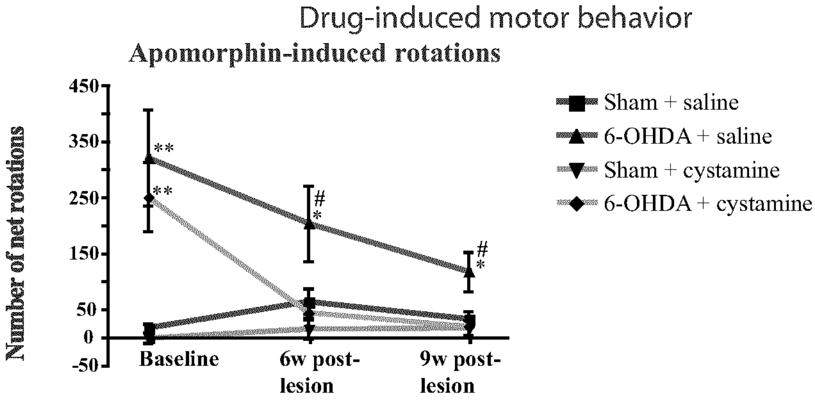


Figure 11a

Spontaneus motor behaviors

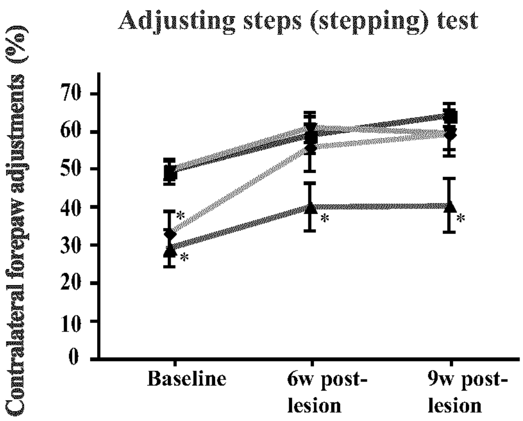


Figure 11b

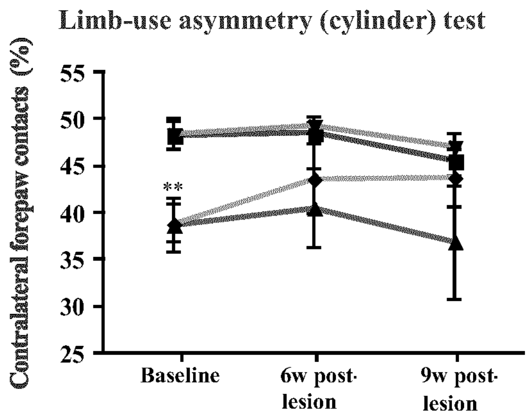


Figure 11c

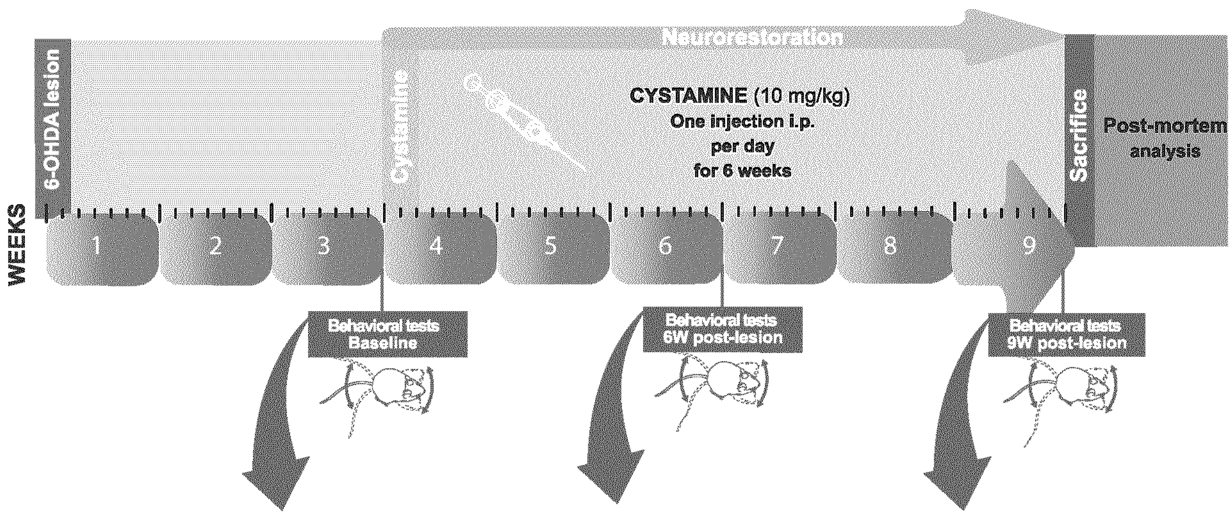


Figure 11d

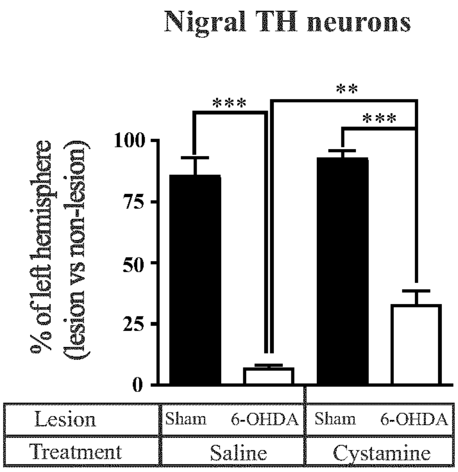


Figure 12a

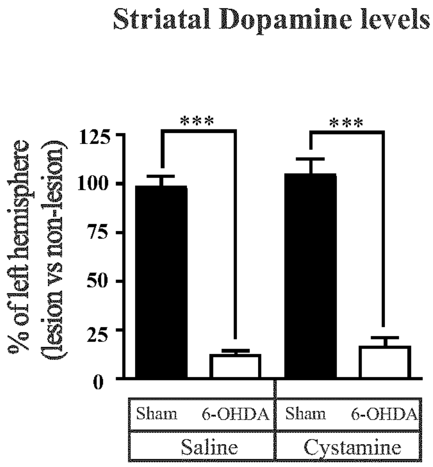


Figure 12b

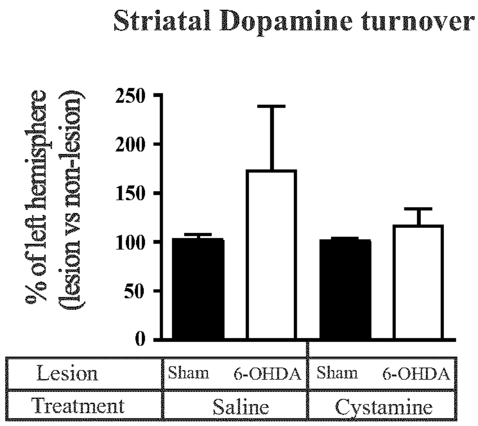


Figure 12c

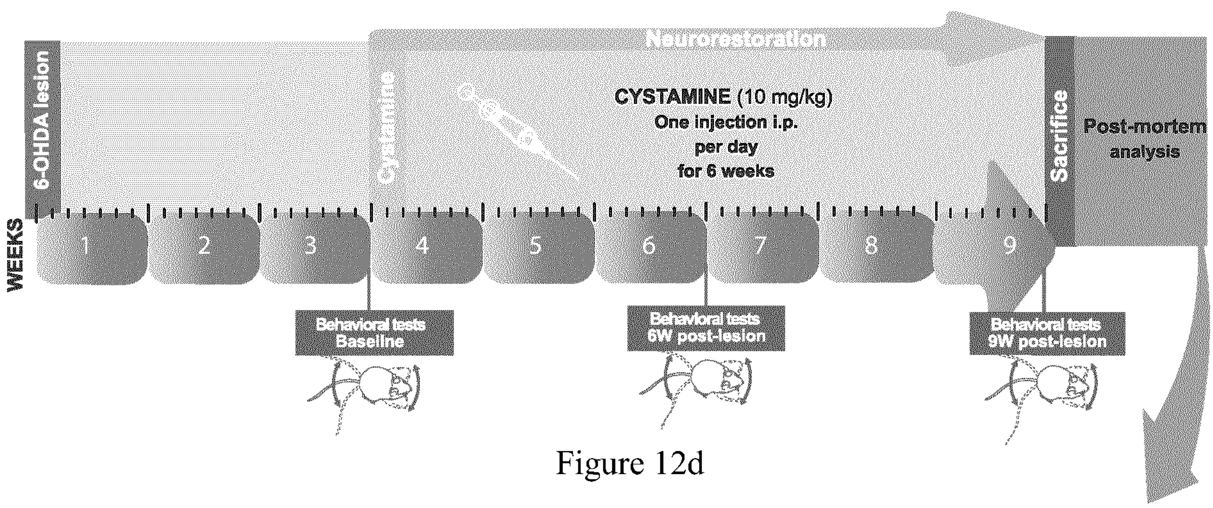


Figure 12d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090076166 A

### Non-patent literature cited in the description


- **GOETZ CG ; TILLEY BC ; SHAFTMAN SR ; STEBBINS GT ; FAHN S ; MARTINEZ-MARTIN P ; POEWE W ; SAMPAIO C ; STERN MB ; DODEL R et al.** Movement Disorder Society-sponsored revision of the Unified Parkinson's Disease Rating Scale (MDS-UPDRS): scale presentation and clinimetric testing results. *Mov Disord,* 2008, vol. 23, 2129-2170
- **HOEHN M M ; YAHR M D.** Parkinsonism: onset, progression and mortality. *Neurology,* 1967, vol. 17, 427-42
- **BERGE et al.** *Pharmaceutical Salts, J. of Phar. Sci.,* January 1977, vol. 66 (1), 1-19
- Handbook of Chemistry and Physics
- **THOMAS SORRELL.** Organic Chemistry. University Science Books, 1999
- March's Advanced Organic Chemistry. John Wiley & Sons, 2001
- **ALLEN P.** Allen brain atlas. Allen Institute for Brain Science, 2008
- **ANDEN, N.E. ; DAHLSTROM, A. ; FUXE, K. ; LARSSON, K.** Functional role of the nigrostriatal dopamine neurons. *Acta Pharmacol. Toxicol.,* 1966, vol. 24, 236-274
- **BACQ Z. M. ; BEAUMARIAGE M. L.** Radioprotective action of cysteamine and cystamine in mice as a function of the period of time between injection of the protector and the start of X-Ray irradiation. *Arch. Int. Pharmacodyn. Ther,* 1965, vol. 153, 457-459
- **BEAUDRY G ; LANGLOIS MC ; WEPPE I ; ROUILLARD C ; LEVESQUE D. CONTRASTING.** patterns and cellular specificity of transcriptional regulation of the nuclear receptor nerve growth factor-inducible B by haloperidol and clozapine in the rat forebrain. *J Neurochem,* 2000, vol. 75, 1694-702
- **BENRABH H. ; BOURRE J. M. ; LEFAUCONNIER J. M.** Taurine transport at the blood-brain barrier: an in vivo brain perfusion study. *Brain Res.,* 1995, vol. 692, 57-65
- **BJORKLUND, A. ; ROSENBLAD, C. ; WINKLER, C. ; KIRIK, D.** Studies on neuroprotective and regenerative effects of GDNF in a partial lesion model of Parkinson's disease. *Neurobiol Dis,* 1997, vol. 4, 186-200
- **BLUME SR ; CASS DK ; TSENG KY.** Stepping test in mice: a reliable approach in determining forelimb akinesia in MPTP-induced Parkinsonism. *Exp Neurol,* 2009, vol. 219, 208-11
- **BORRELL-PAGES M. ; CANALS J. M. ; CORDELIERES F. P. et al.** Cystamine and cysteamine increase brain levels of BDNF in Huntington disease via HSJ1b and transglutaminase. *J. Clin. Invest,* 2006, vol. 116, 1410-1424
- **BOUCKENOOGHE T. ; REMACLE C. ; REUSENS B.** Is taurine a functional nutrient?. *Curr: Opin. Clin. Nutr. Metab. Care,* 2006, vol. 9, 728-733
- **BOURASSET F. ; CISTERNINO S. ; TEMSAMANI J. ; SCHERRMANN J. M.** Evidence for an active transport of morphine-6-beta-d-glucuronide but not P-glycoprotein-mediated at the blood-brain barrier. *J. Neurochem.,* 2003, vol. 86, 1564-1567
- **BOUSQUET M ; GIBRAT C ; SAINT-PIERRE M ; JULIEN C ; CALON F ; CICCHETTI F.** Modulation of brain-derived neurotrophic factor as a potential neuroprotective mechanism of action of omega-3 fatty acids in a parkinsonian animal model. *Prog Neuropsychopharmacol Biol Psychiatry,* 13 November 2009, vol. 33 (8), 1401-8
- **BOUSQUET M. ; GIBRAT C. ; OUELLET M. ; ROUILLARD C. ; CALON F. ; CICCHETTI F.** Cystaminemetabolism and brain transport properties: clinical implications for neurodegenerative diseases. *J. Neurochem.,* 2010, vol. 114, 1651-1658
- **BOUSQUET M ; GUE K ; EMOND V ; JULIEN P ; KANG JX ; CICCHETTI F ; CALON F.** Transgenic conversion of omega-6 into omega-3 fatty acids in a mouse model of Parkinson's disease. *J Lipid Res.,* February 2011, vol. 52 (2), 263-71
- **CALON F. ; MORISSETTE M. ; GOULET M. ; GRONDIN R. ; BLANCHET P. J. ; BEDARD P. J. ; DI PAOLO T.** Chronic DI and D2 dopaminomimetic treatment of MPTP-denervated monkeys: effects on basal ganglia GABA(A)/benzodiazepine receptor complex and GABA content. *Neurochem. Int,* 1999, vol. 35, 81-91

- **CALON F. et al.** Effect of MPTP-induced denervation on basal ganglia GABA(B) receptors: correlation with dopamine concentrations and dopamine transporter. *Synapse,* 01 June 2001, vol. 40 (3), 225-34
- **CALON F. et al.** Changes of GABA receptors and dopamine turnover in the postmortem brains of parkinsonians with levodopa-induced motor complications. *Mov Disord.,* March 2003, vol. 18 (3), 241-53
- **CAVALLINI D. ; SCANDURRA R. ; DEMARCO C.** The enzymatic oxidation of cysteamine to hypotaurine in the presence of sulfide. *J. Bio. Chem.,* 1963, vol. 238, 2999-3005
- **COLOSO R. M. ; HIRSCHBERGER L. L. ; DOMINY J. E. ; LEE J. I. ; STIPANUK M. H.** Cysteamine dioxygenase: evidence for the physiological conversion of cysteamine to hypotaurine in rat and mouse tissues. *Adv. Exp. Med. Biol.,* 2006, vol. 583, 25-36
- **COSTANTINI LC ; COLE D ; CHATURVEDI P ; ISACSON O.** Immunophilin ligands can prevent progressivedopaminergic degeneration in animal models of Parkinson's disease. *Eur J Neurosci. Mar.*
- **COSSETTE M ; PARENT A ; LEVESQUE D.** Tyrosine hydroxylase-positive neurons intrinsic to the human striatum express the transcription factor Nurr1. *Eur J NeuroSci.,* 2004, vol. 20, 2089-95
- **DAGENAIS C. ; ROUSSELLE C. ; POLLACK G. M. ; SCHERRMANN J. M.** Development of an in situ mouse brain perfusion model and its application to mdrla P-glycoprotein-deficient mice. *J. Cereb. Blood Flow Metab.,* 2000, vol. 20, 381-386
- **DEDEOGLU A. ; KUBILUS J. K. ; JEITNER T. M. et al.** Therapeutic effects of cystamine in a murine model of Huntington's disease. *J. Neurosci.,* 2002, vol. 22, 8942-8950
- **DOHIL R. ; FIDLER M. ; GANGOITI J. A. ; KASKEL F. ; SCHNEIDER J. A. ; BARSHOP B. A.** Twice-daily cysteamine bitartrate therapy for children with cystinosis. *J. Pediatr.,* 2009, vol. 156, 71-75
- **DOMINY J. ; ELLER S. ; DAWSON R. JR.** Building biosynthetic schools: reviewing compartmentation of CNS taurine synthesis. *Neurochem. Res.,* 2004, vol. 29, 97-103
- **DOMINY J. E. JR ; SIMMONS C. R. ; HIRSCHBERGER L. L. ; HWANG J. ; COLOSO R. M. ; STIPANUK M. H.** Discovery and characterization of a second mammalian thiol dioxygenase, cysteamine dioxygenase. *J. Biol. Chem.,* 2007, vol. 282, 25189-25198
- **DUBINSKY R. ; GRAY C.** CYTE-I-HD: phase I dose finding and tolerability study of cysteamine (Cystagon) in Huntington's disease. *Mov. Disord.,* 2006, vol. 21, 530-533
- **EWETZ L. ; SORBO B.** Characteristics of the cysteinesulfinate-forming enzyme system in rat liver. *Biochim. Biophys. Acta,* 1966, vol. 128, 296-305
- **FOX J. H. ; BARBER D. S. ; SINGH B. et al.** Cystamine increases L-cysteine levels in Huntington's disease transgenic mouse brain and in a PC12 model of polyglutamine aggregation. *J. Neurochem.,* 2004, vol. 91, 413-422
- **FRENCH E. D. ; VEZZANI A. ; WHETSELL W. O. JR. ; SCHWARCZ R.** Anti-excitotoxic actions of taurine in the rat hippocampus studied in vivo and in vitro. *Adv. Exp. Med. Biol.,* 1986, vol. 203, 349-362
- **GIBRAT C. ; BOUSQUET M. ; SAINT-PIERRE M. ; LEVESQUE D. ; CALON F. ; ROUILLARD C. ; CICCHETTI F.** Cystamine prevents MPTP-induced toxicity in young adult mice via the up-regulation of the brain-derived neurotrophic factor. *Prog. Neuropsychopharmacol. Biol. Psychiatry,* 2010, vol. 34, 193-203
- **GIBRAT C ; SAINT-PIERRE M ; ROUILLARD C ; CICCHETTI F.** Neuroprotective properties of cystamine in acute vs chronic models of Parkinson's disease. *The Canadian Journal of Neurological Sciences,* 2007, vol. 3, S42
- **GIBRAT C ; BOUSQUET M ; SAINT-PIERRE M ; LÉVESQUE D ; CALON F ; ROUILLARD C ; CICCHETTI F.** Neuroprotective mechanisms of cystamine in a murine model of Parkinson's disease. *Parkinsonism and Related Disorders,* 2009, vol. 15S2, S29-S199
- **GIBRAT C ; SAINT-PIERRE M ; BOUSQUET M ; LEVESQUE D ; ROUILLARD C ; CICCHETTI F.** Differences between subacute and chronic MPTP mice models: investigation of dopaminergic neuronal degeneration and alpha-synuclein inclusions. *J Neurochem,* 2009, vol. 109, 1469-82
- **GREEN H.** Human genetic diseases due to codon reiteration: relationship to an evolutionary mechanism. *Cell,* 1993, vol. 74, 955-956
- **HUXTABLE R. J.** Physiological actions of taurine. *Physiol. Rev.,* 1992, vol. 72, 101-163
- **LANCU R ; MOHAPEL P ; BRUNDIN P ; PAUL G.** Behavioral characterization of a unilateral 6-OHDA-lesion model of Parkinson's disease in mice. *Behav Brain Res,* 2005, vol. 162, 1-10
- **JEITNER T. M. ; DELIKATNY E. J. ; AHLQVIST J. ; CAPPER H. ; COOPER A. J.** Mechanism for the inhibition of transglutaminase 2 by cystamine. *Biochem. Pharmacol.,* 2005, vol. 69, 961-970
- **KARPUJ M. V. ; BECHER M. W. ; SPRINGER J. E. ; CHABAS D. ; YOUSSEF S. ; PEDOTTI R. ; MITCHELL D. ; STEINMAN L.** Prolonged survival and decreased abnormal movements in transgenic model of Huntington disease, with administration of the transglutaminase inhibitor cystamine. *Nat. Med.,* 2002, vol. 8, 143-149

- **LAPOINTE N ; ST-HILAIRE M ; MARTINOLI MG ; BLANCHET J ; GOULD P ; ROUILLARD C.** Rotenone induces non-specific central nervous system and systemic toxicity. *FASEB J.,* 2004, vol. 18, 717-9
- **LEE J. I. ; LONDONO M. ; HIRSCHBERGER L. L. ; STIPANUK M. H.** Regulation of cysteine dioxygenase and gamma-glutamylcysteine synthetase is associated with hepatic cysteine level. *J Nutr. Biochem.,* 2004, vol. 15, 112-122
- **LEIN ES ; HAWRYLYCZ MJ et al.** Genome-wide atlas of gene expression in the adult mouse brain. *Nature,* 2007, vol. 445, 168-76
- **LEONARDI R. ; ZHANG Y. M. ; ROCK C. O. ; JACKOWSKI S.** Coenzyme A: back in action. *Prog. Lipid Res.,* 2005, vol. 44, 125-153
- **LINDNER MD ; WINN SR ; BAETGE EE ; HAMMANG JP ; GENTILE FT ; DOHERTY E et al.** Implantation of encapsulated catecholamine and GDNF-producing cells in rats with unilateral dopamine depletions and parkinsonian symptoms. *Exp Neurol,* 1995, vol. 132 (62-76.), 690
- **LUNDBLAD M ; PICCONI B ; LINDGREN H ; CENCI MA.** A model of l-DOPA-induced dyskinesia in 6-hydroxydopamine lesioned mice: relation to motor and cellular parameters of nigrostriatal function. *Neurobiol Dis,* 2004, vol. 16, 110-23
- **MARTIGNONI M. ; GROOTHUIS G. M. ; DE KANTER R.** Species differences between mouse, rat, dog, monkey and human CYP-mediated drag metabolism, inhibition and induction. *Expert Opin. Drug Metab. Toxicol.,* 2006, vol. 2, 875-894
- **MURAMATSU M. ; KAKITA K. ; NAKAGAWA K. ; KURIYAMA K.** A modulating role of taurine on release of acetylcholine and norepinephrine from neuronal tissues. *Jpn. J. Pharmacol.,* 1978, vol. 28, 259-268
- **OJA S. S. ; SARANSAARI P.** Taurine as osmoregulator and neu-romodulator in the brain. *Metab. Brain Dis.,* 1996, vol. 11, 153-164
- **OLDENDORF W. H. ; SZABO J.** Amino acid assigmnent to one of three blood-brain barrier amino acid carriers. *Am. J. Physiol.,* 1976, vol. 230, 94-98
- **OUELLET M. ; EMOND V. ; CHEN C. T. ; JULIEN C. ; BOURASSET F. ; ODDO S. ; LAFERLA E ; BAZINET R. P. ; CALON F.** Diffusion of docosahexaenoic and eicosapentaenoic acids through the blood-brain barrier: and in situ cerebral perfusion study. *Neurochem. Int.,* 2009, vol. 55, 476-482
- **PASANTES-MORALES H. ; ARZATE N. E. ; CRUZ C.** The role of taurine in nervous tissue: its effects on ionic fluxes. *Adv Exp Med Biol,* 1981, vol. 139, 273-292
- **PINTO J. T. ; VAN RAAMSDONK J. M. ; LEAVITT B. R. ; HAYDEN M. R. ; JEITNER T. M. ; THALER H. T. ; KRASNIKOV B. F. ; COOPER A. J.** Treatment of YAC128 mice and their wild-type littermates with cystamine does not lead to its accumulation in plasma or brain: implications for the treatment of Huntington disease. *J Neurochem,* 2005, vol. 94, 1087-1101
- **PINTO J. T. ; KHOMENKO T. ; SZABO S. ; MCLAREN G. D. ; DENTON T. T. ; KRASNIKOV B. F. ; JEITNER T. M. ; COOPER A. J.** Measurement of sulfur-containing compounds involved in the metabolism and transport of cysteamine and cystamine. Regional differences in cerebral metabolism. *J Chromatogr B Analyt Technol Biomed Life Sci,* 2009, vol. 877, 3434-3441
- **PITARI G. ; MAURIZI G. ; FLATI V. ; URSINI C. L. ; SPERA L. ; DUPRE S. ; CAVALLINI D.** Enzymatic synthesis of S-aminoethyl-L-cysteine from pantetheine. *Biochim Biophys Acta,* 1992, vol. 1116, 27-33
- **SALLER C. F. ; CZUPRYNA M. J.** gamma-Aminobutyric acid, glutamate, glycine and taurine analysis using reversed-phase high-performance liquid chromatography and ultraviolet detection of dansyl chloride derivatives. *J Chromatogr,* 1989, vol. 487, 167-172
- **SANTA T. ; AOYAMA C. ; FUKUSHIMA T. ; IMAI K. ; FUNATSU T.** Suppression of thiol exchange reaction in the determination of reduced-form thiols by high-performance liquid chromatography with fluorescence detection after derivatization with fluorogenic benzofurazan reagent, 7-fluoro-2,1,3-benzoxadiazole-4-sulfonate and 4-aminosulfonyl-7-fluoro-2,1,3-benzoxadiazole. *Biomed Chromatogr,* 2006, vol. 20, 656-661
- **SCHALLERT T ; FLEMING SM ; LEASURE JL ; TILLERSON JL ; BLAND ST.** CNS plasticity and assessment of forelimb sensorimotor outcome in unilateral rat models of stroke, cortical ablation, parkinsonism and spinal cord injury. *Neuropharmacology,* 2000, vol. 39, 777-87
- **SERSHEN H. ; LAJTHA A.** Inhibition pattern by analogs indicates the presence of ten or more transport systems for amino acids in brain cells. *J Neurochem,* 1979, vol. 32, 719-726
- **STACK E. C. ; FERRO J. L. ; KIM J. ; DEL SIGNORE S. J. ; GOODRICH S. ; MATSON S. ; HUNT B. B. ; CORMIER K. ; SMITH K. ; MATSON W. R.** Therapeutic attenuation of mitochondrial dysfunction and oxidative stress in neurotoxin models of Parkinson's disease. *Biochim Biophys Acta,* 2008, vol. 1782, 151-162
- **SUN L. ; XU S. ; ZHOU M. ; WANG C. ; WU Y. ; CHAN P.** Effects of cysteamine on MPTP-induced dopaminergic neurodegeneration in mice. *Brain Res.,* 2010, vol. 1335, 74-82

- **TILLERSON JL ; COHEN AD ; PHILHOWER J ; MILLER GW ; ZIGMOND MJ ; SCHALLERT T.** Forced limb-use effects on the behavioral and neurochemical effects of 6-hydroxydopamine. *J Neurosci,* 2001, vol. 21, 4427-35
- **TREMBLAY M. E. ; SAINT-PIERRE M. ; BOURHIS E. ; LEVESQUE D. ; ROUILLARD C. ; CICCHETTI F.** Neuroprotective effects of cystamine in aged parkinsonian mice. *Neurobiol Aging,* 2006, vol. 27, 862-870
- **UNGERSTEDT, U.** 6-Hydroxydopamine-induced degeneration of central monoamine neurons. *Eur. J. Pharmacol.,* 1968, vol. 5, 5107-5110
- **WADE L. A. ; KATZMAN R.** Synthetic amino acids and the nature of L-DOPA transport at the blood-brain barrier. *J Neurochem,* 1975, vol. 25, 837-842
- **WADE L. A. ; BRADY H. M.** Cysteine and cystine transport at the blood-brain barrier. *J Neurochem,* 1981, vol. 37, 730-734
- **WANG X. ; SARKAR A. ; CICCHETTI F. ; YU M. ; ZHU A. ; JOKIVARSI K. ; SAINT-PIERRE M. ; BROWNELL A. L.** Cerebral PET imaging and histological evidence of transglutaminase inhibitor cystamine induced neuroprotection in transgenic R6/2 mouse model of Huntington's disease. *J Neurol Sci,* 2005, vol. 231, 57-66
- **ZETTERSTROM ; ROLF H. LUDMILA SOLOMIN ; LOTTIE JANSSON ; BARRY J. HOFFER ; LARS OLSON ; THOMAS PERLMANN.** Dopamine neuron agenesis in Nurr1-deficient mice. *Science,* 11 April 1997, vol. 276 (5310), 248-50